# FASCICULE DE BREVET EUROPEEN

(11) **EP 3 278 095 B1**
(45) Date de publication et mention de la délivrance du brevet: **01.12.2021**
(21) Numéro de dépôt: 16717283.2
(22) Date de dépôt: 01.04.2016
(51) Int. Cl.: G01N 27/48, C12Q 1/04

(54) **NOUVELLE METHODE POUR DETECTER LA PRESENCE DE BACTERIES PRODUCTRICES DE BETA-LACTAMASES**
NEUARTIGES VERFAHREN ZUM NACHWEIS DER PRÄSENZ VON BETA-LACTAMASE-PRODUZIERENDEN BAKTERIEN
NOVEL METHOD FOR DETECTING THE PRESENCE OF BETA-LACTAMASE-PRODUCING BACTERIA

(30) Priorité: 03.04.2015 FR 1552928
(43) Date de publication de la demande: 07.02.2018
(73) Titulaire: Université de Bourgogne, 21078 Dijon Cedex (FR); Centre Hospitalier Universitaire De Dijon, 21079 Dijon Cedex (FR); Institut national de recherche pour l'agriculture, l'alimentation et l'environnement, 75007 Paris (FR)
(72) Inventeur: DEQUAIRE-ROCHELET, Murielle, 21000 Dijon (FR); HARTMANN, Alain, 21000 Dijon (FR); NEUWIRTH, Catherine, 21000 Dijon (FR); CHANTEMESSE, Benoit, 21000 Dijon (FR)
(74) Mandataire: IPAZ
(86) Numéro de dépôt international: PCT/EP2016/057284
(87) Numéro de publication internationale: WO 2016/156605

(56) Documents cités:
- WO-A1-2009/051838
- WO-A1-2013/072494
- US-A1- 2013 089 883
- MITTELMANN ADRIAN SCHWARTZ ET AL: "Amperometric quantification of total coliforms and specific detection of Escherichia coli", ANALYTICAL CHEMISTRY, AMERICAN CHEMICAL SOCIETY, US, vol. 74, no. 4, 1 février 2002 (2002-02-01), pages 903-907, XP009095590, ISSN: 0003-2700, DOI: 10.1021/AC0156215
- LUÍS MOREIRA GONÇALVES ET AL: "Penicillinase-based amperometric biosensor for penicillin G", ELECTROCHEMISTRY COMMUNICATIONS, vol. 38, 1 janvier 2014 (2014-01-01), pages 131-133, XP055249353, NL ISSN: 1388-2481, DOI: 10.1016/j.elecom.2013.11.022
- Yiting Chen ET AL: "Rapid hydrolysis and Electrochemical Detection of Cephalexin at a Heated Glassy Carbon Electrode", Int. J. Electrochem. Sci. International Journal, 1 janvier 2012 (2012-01-01), pages 7948-7959, XP055249465, Extrait de l'Internet: URL:http://www.electrochemsci.org/papers/v ol7/7097948.pdf [extrait le 2016-02-11]
- HIDEAKI HANAKI ET AL: "Substrate specificity of HMRZ-86 for Î-lactamases, including extended-spectrum Î-lactamases (ESBLs)", JOURNAL OF INFECTION AND CHEMOTHERAPY ; OFFICIAL JOURNAL OF THE JAPANESE SOCIETY OF CHEMOTHERAPY AND THE JAPANESE ASSOCIATION FORINFECTIOUS DISEASES, SPRINGER-VERLAG, TO, vol. 13, no. 6, 25 décembre 2007 (2007-12-25), pages 390-395, XP019567039, ISSN: 1437-7780
- J. PIRES ET AL: "Blue-Carba, an Easy Biochemical Test for Detection of Diverse Carbapenemase Producers Directly from Bacterial Cultures", JOURNAL OF CLINICAL MICROBIOLOGY, vol. 51, no. 12, 9 octobre 2013 (2013-10-09), pages 4281-4283, XP055281119, US ISSN: 0095-1137, DOI: 10.1128/JCM.01634-13
- Anonymous: "[beta] CARBA Test 25: Rapid detection of carbapenemase-producing Enterobacteriaceae strains Contents", , 31 mai 2015 (2015-05-31), XP055281173, Extrait de l'Internet: URL:http://www.bio-rad.com/webroot/web/pdf /inserts/CDG/en/68260_881159_EN.pdf [extrait le 2016-06-16]
- Bio-Rad: "Beta Carba Test", , 19 août 2015 (2015-08-19), XP055281201, Extrait de l'Internet: URL:http://www.bio-rad.com/webroot/web/pdf /WWMSDS/CMD/D/D_D_68260.pdf [extrait le 2016-06-16]

## Description

La présente invention concerne une méthode pour détecter, dans un échantillon ou un milieu de culture, la présence de bactéries résistantes aux bêta-lactamines via leur capacité à produire des bêta-lactamases.

Les bêta-lactamines, qui comprennent les pénicillines, les monobactames, les céphalosporines et les carbapénèmes, sont des antibiotiques qui agissent par inhibition de la synthèse du peptidoglycane. Ce sont des molécules utilisées fréquemment en traitement de première intention des infections graves tant en pratique de ville qu'en médecine hospitalière. Les bêta-lactamases sont des enzymes qui hydrolysent le noyau bêta-lactame des bêta-lactamines. C'est le mécanisme de résistance aux bêta-lactamines le plus fréquent. Les bêta-lactamases sont classées selon Ambler (Ambler, R.P. Philos. Trans. R. Soc. London Ser B, 1980, 289, 321-331) en 4 groupes :
A : pénicillinases, dont les bêta-lactamases à large spectre étendu BLSEs
B : métallo-enzymes
C : céphalosporinases
D : oxacillinases

Chez les entérobactéries, la production de bêta-lactamases à spectre étendu (désignées ci-après par BLSEs) ou de céphalosporinases hyperproduites est actuellement le principal mécanisme de résistance aux céphalosporines de 3^{ème} génération (désignées ci-après par C3G), telles que le céfotaxime, la ceftriaxone ou la ceftazidime, qui sont les oxyimino-céphalosporines. Le céfépime, qui est la seule céphalosporine de 4^{ème} génération (désignée ci-après par C4G), est quant à lui hydrolysé par les BLSEs mais habituellement pas par les céphalosporinases. Les pénicillines sont également hydrolysées, mais pas les carbapénèmes. L'activité des BLSEs peut être inhibée par l'acide clavulanique, le tazobactam ou le sulbactam (inhibiteurs de pénicillinases). L'activité des céphalosporinases peut être inhibée par la cloxacilline. Les carbapénémases (enzymes pouvant appartenir aux classes A, B et D selon Ambler) sont des enzymes qui hydrolysent les carbapénèmes mais également le plus souvent les pénicillines et les céphalosporines.

La détection précise et rapide des bactéries résistantes aux céphalosporines de 3^{ème} génération, productrices de BLSEs ou de céphalosporinases hyperproduites, ou de carbapénémases, est cruciale pour la prise en charge des patients. Elle doit conduire à la mise en place d'un traitement antibiotique adapté. En l'absence de production d'enzymes hydrolysant les C3G, ces dernières peuvent être utilisées en première intention, alors que dans le cas contraire un recours aux carbapénèmes s'avère nécessaire. Dans ce cas, il est également nécessaire de déterminer si ces bactéries produisent des carbapénèmases.

Jusqu'ici, la technique classique utilisée pour détecter les bactéries produisant des bêta-lactamases est basée sur la mise en œuvre d'un antibiogramme et sur la réalisation du test de double synergie qui permet de mettre en évidence la restauration de la sensibilité aux C3G en présence d'inhibiteur de pénicillinases. Malgré son efficacité, la mise en oeuvre de cette technique nécessite une étape préalable de mise en culture et d'isolement des bactéries, ce qui implique un délai d'au moins 24 heures avant que la production de bêta-lactamases soit détectable.

La présence de bactéries productrices de bêta-lactamases également peut être mise en évidence par des méthodes de biologie moléculaire, par exemple en détectant par PCR et/ou séquençage les gènes codant pour les bêta-lactamases. Bien qu'elles puissent permettre une caractérisation spécifique des gènes codant par exemple les BLSEs, ces techniques de laboratoire sont coûteuses et assez longues à mettre en oeuvre à cause de l'extraction préalable de l'ADN d'échantillons complexes.

Il existe donc un besoin urgent dans le domaine de la santé de développer une méthode efficace, rapide et de faible coût pour détecter la présence des bactéries productrices des bêta-lactamases, afin de détecter la présence des souches bactériennes résistantes aux bêta-lactamines.

Nordmann et al. (J.Clin. Microbiol., 2012, 50, 3016-3022) décrivent une méthode de détection des bactéries produisant des BLSEs qui implique l'utilisation du céfotaxime et d'un indicateur coloré de pH. Cette méthode repose sur le fait que l'hydrolyse du noyau bêta-lactame du céfotaxime conduit à la formation d'une fonction acide carboxylique et donc à l'acidification du milieu réactionnel. Le changement de pH du milieu est visualisé grâce à l'utilisation d'un indicateur coloré.

Malgré son efficacité et sa rapidité, cette méthode nécessite préalablement une étape contraignante d'isolement des souches bactériennes.

Une autre approche colorimétrique consiste à utiliser la nitrocéfine, une céphalosporine chromogène, qui peut être hydrolysée par toutes les bêta-lactamases, provoquant ainsi le changement de couleur du jaune au rouge (O'Callaghan et al., Antimicrob. Agents Chemother., 1972, 1, 283-288). Le schéma 1 ci-après illustre l'hydrolyse du noyau bêta-lactame de la nitrocéfine par une bêta-lactamase. Plus récemment, une autre céphalosporine chromogène nommée HMRZ-86 (Hanaki et al., Antimicrob. Agents Chemother., 2004, 53, 888-889) a été proposée pour détecter selon le même principe les souches bactériennes résistantes aux C3G.

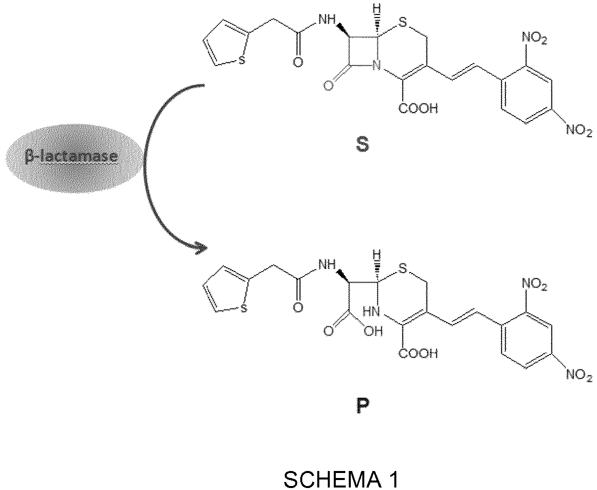

Cependant, cette méthode nécessite à nouveau l'utilisation de souches bactériennes préalablement isolées pour permettre la détection colorimétrique. Cette méthode n'est pas directement utilisable sur des échantillons complexes présentant une coloration qui peut interférer avec la détection colorimétrique de l'hydrolyse de la nitrocéfine. Là encore, cette méthode impose un délai d'au moins 24 heures entre le prélèvement d'un échantillon clinique et le rendu du résultat.

Ces dernières années, la technique de spectrométrie de masse MALDI-TOF a également été proposée pour détecter les produits d'hydrolyse des bêta-lactamines après incubation de ces dernières en présence de souches productrices de bêta-lactamases (Hrabàk et al. J. Clin. Microbiol. 2011, 49, 3222-3227 ; Sparbier et al., J. Clin. Microbiol., 2012, 50, 927-937). Toutefois, cette technique de laboratoire, qui doit aussi être mise en oeuvre avec des souches préalablement isolées, reste coûteuse et nécessite du personnel qualifié pour l'interprétation des spectres de masse.

Par ailleurs, Gonçalves et al. (Electrochemistry Communication 38 (2014) 131-133) présentent un biocapteur ampérométrique pour la détection de la pénicilline G par la mesure ampérométrique de la variation de pH du milieu liée à l'hydrolyse par pénicillinase de la pénicilline G en acide pénicilloïque. Comme la pénicilline G et l'acide pénicilloïque ne sont pas électroactifs, la mesure ampérométrique est effectuée en présence de cobalt qui joue le rôle de médiateur rédox.

Chen et al. (Int. J. Electrochem. Sci., 7 (2012) 7948-7959) décrivent une méthode pour détecter électrochimiquement la céphalexine après son hydrolyse obtenue suite au chauffage à 70°C d'une solution alcaline de céphalexine privée d'oxygène et à l'obscurité. Les produits d'hydrolyse de la céphalexine (coupure de la liaison C-C=O du cycle β-lactame) décrits dans ce document ont été produits dans des conditions extrêmes bien spécifiques et ne peuvent pas être obtenus en incubant la céphalexine avec une bêta-lactamase, qui, elle, coupe le cycle β-lactame au niveau de la liaison amide.

A ce jour, l'art antérieur ne décrit aucun substrat des bêta-lactamases ou aucun produit résultant de l'hydrolyse par une bêta-lactamase ayant des propriétés électroactives.

Or les Inventeurs ont découvert que certains substrats des bêta-lactamases et/ou leur produit hydrolyse par une bêta-lactamase présentent des propriétés électroactives.

Dans ce contexte, l'objectif de l'invention est donc de mettre à disposition une méthode électrochimique capable de déterminer rapidement et efficacement la présence de bactéries productrices de bêta-lactamases dans un échantillon sans isoler au préalable les souches bactériennes.

L'invention porte sur une méthode pour déterminer *in vitro* la présence de bactéries produisant des bêta-lactamases dans un échantillon susceptible de contenir lesdites bactéries, comprenant les étapes suivantes:
(a) incuber ledit échantillon dans un milieu contenant un substrat des bêta-lactamases possédant des propriétés électrochimiques, notamment une bêta lactamine possédant des propriétés électrochimiques, plus particulièrement une céphalosporine, en tant que substrat des bêta-lactamases,
(b) appliquer un moyen d'analyse électrochimique pour déterminer la présence des bactéries produisant des bêta-lactamases.

L'étape de détermination de la présence de bactéries productrices de beta-lactamases est effectuée par détection électrochimique de la présence du substrat de beta-lactamase hydrolysé.

L'invention repose sur le fait que certains substrats des bêta-lactamases après avoir été hydrolysés par une bêta-lactamase peuvent être détectés électrochimiquement.

La méthode de la présente invention est définie dans la revendication 1.

Notamment, l'hydrolyse par une bêta-lactamase du noyau bêta-lactame d'une bêta-lactamine peut être détectée électrochimiquement.

Dans le cadre de la présente invention, une bêta-lactamine possédant des propriétés électrochimiques est notamment une céphalosporine, plus particulièrement la nitrocéfine ou le composé HMRZ-86 (Isomère E de l'acide (6R,7R)-trifluoroacétate 7-[[2-(2-amino-4-thiazolyl)-2-[(1-carboxy-1-méthyléthoxy)imino]acétyl]amino]-1-aza-3-[2-(2,4-dinitrophényl)éthényl]-8-oxo-5-thiabicyclo[4.2.0]oct-2-ène-2-carboxylique).

Dans le cadre de la présente invention, un tel substrat des bêta-lactamases possédant des propriétés électrochimiques peut être aussi le substrat (réactif R2) du kit β CARBA^{™} commercialisé par Bio-rad.

La nitrocéfine peut être hydrolysée par tous les types de β-lactamases tandis que le composé HMRZ-86 est uniquement dégradé par les bactéries résistantes aux C3G ; c'est-à-dire productrices de BLSEs, de céphalosporines hyperproduites et de carbapénémases. Le substrat (réactif R2) du kit β CARBA^{™} est hydrolysé uniquement par les bactéries produisant des carbapénémases.

Les Inventeurs ont mis en évidence pour la première fois que les propriétés électrochimiques de certains substrats des bêta-lactamases, notamment certaines bêta-lactamines, telles que la nitrocéfine ou le composé HMRZ-86, sont bien distinctes de celles de ces substrats hydrolysés. Par exemple, la nitrocéfine hydrolysée est un produit électroactif capable de générer un courant d'oxydation anodique spécifique et mesurable par une méthode électrochimique. L'oxydation de la nitrocéfine hydrolysée génère un courant anodique dans un intervalle de potentiels différent de celui dans lequel apparaît ledit courant anodique correspondant à l'oxydation de la nitrocéfine. Ainsi, le courant anodique spécifique à la nitrocéfine hydrolysée peut être utilisé comme la réponse analytique indiquant la présence de la nitrocéfine hydrolysée par une bêta-lactamase.

Contrairement aux méthodes déjà développées dans l'art antérieur qui nécessitent une mise en culture des bactéries pendant une durée d'au moins 24 heures au préalable, la méthode décrite dans l'invention permet de détecter la présence ou l'absence de bactéries produisant des bêta-lactamases après seulement quelques heures d'incubation de l'échantillon, notamment de 1 à 4 heures, avant la mise en œuvre de la mesure électrochimique.

A titre d'exemple, le temps d'incubation d'un échantillon avec un milieu contenant la nitrocéfine est typiquement de 5 à 30 minutes.

Un milieu contenant un substrat des bêta-lactamases possédant des propriétés électrochimiques peut être un milieu tamponné dans lequel est ajouté un substrat électroactif des bêta-lactamases. Ledit milieu tamponné peut être, à titre d'exemple, un tampon phosphate de pH 7 et de concentration de 100 mM.

On entend par « bêta-lactamases » une famille d'enzymes capables d'hydrolyser le noyau β-lactame d'une β-lactamine. Les pénicillines, les céphalosporines, les monobactames et les carbapénèmes sont les quatre familles de β-lactamines.

Selon leurs propriétés catalytiques et leur affinité pour certains substrats ainsi que leurs sensibilités à certains types d'inhibiteurs de bêta-lactamases, les principales bêta-lactamases sont les suivantes : les pénicillinases, les céphalosporinases inductibles, les céphalosporinases hyperproduites, les bêta-lactamases à spectre étendu, et les carbapénèmases.

On entend par « pénicillinases » les enzymes qui hydrolysent les amino-, les carboxy- et les uréidopénicillines. Elles sont inhibées par des inhibiteurs de pénicillinases que sont l'acide clavulanique, le tazobactam et le sulbactam.

On entend par « céphalosporinases inductibles » les enzymes qui hydrolysent les amino-pénicillines et les céphalosporines de 1^{ère} et/ou 2^{ème} génération. Elles sont insensibles à l'action des inhibiteurs de pénicillinases ; par contre, elles sont inhibées par la cloxacilline. L'expression d'un gène codant une céphalosporinase peut être induite par un inducteur, qui peut être notamment une bêta-lactamine.

On entend par « céphalosporinases hyperproduites » les enzymes qui hydrolysent les aminopénicillines, les carboxy- et les uréidopénicillines, les céphalosporines de 1^{ère}, 2^{ème}, 3^{ème} voire de 4^{ème} génération. Elles sont insensibles à l'action des inhibiteurs de pénicillinases ; par contre, elles sont inhibées par la cloxacilline. L'expression de ces enzymes est déréprimée et conduit à une production en abondance de ces enzymes.

On entend par « bêta-lactamases à spectre étendu (BLSEs) » les enzymes qui hydrolysent les amino-, les carboxy- et les uréido-pénicillines, les céphalosporines de 1^{ère}, 2^{ème}, 3^{ème} et 4^{ème} génération et sont sensibles à l'inhibition des inhibiteurs de pénicillinases.

On entend par « carbapénèmases » les enzymes qui possèdent une activité hydrolytique sur les carbapénèmes mais généralement aussi sur les pénicillines et les céphalosporines de 1^{ère}, 2^{ème}, 3^{ème} et 4^{ème} génération.

Conformément à l'invention, les bêta-lactamases peuvent être produites en particulier par des bactéries à Gram négatif :
i) de la famille des *Enterobacteriaceae,* notamment des genres *Escherichia* (en particulier *Escherichia coli), Klebsiella, Enterobacter, Serratia, Morganella, Proteus, Providencia, Pantoea, Hafnia, Citrobacter, Salmonella, Shigella* et *Yersinia.*
*ii)* non fermentaires, notamment *Pseudomonas* (en particulier *P. aeruginosa), Stenotrophomonas* et *Achromobacter.*
iii) *des Acinetobacter* (en particulier *A. baumannii*)*.*

Dans un mode de réalisation particulier, la susdite méthode de l'invention comprend en outre, avant la mise en oeuvre de l'étape (a), une étape pour concentrer les bactéries présentes dans un échantillon susceptible de les contenir. La concentration des bactéries peut être effectuée, par exemple, par filtration ou centrifugation.

Dans un mode de réalisation particulier, l'étape (a) de la susdite méthode de l'invention est mise en oeuvre dans un milieu contenant un substrat des bêta-lactamases possédant des propriétés électrochimiques, notamment une bêta-lactamine possédant des propriétés électrochimiques, en particulier la nitrocéfine, le composé HMRZ-86 ou le substrat (réactif R2) du kit β CARBA^{™}. L'homme du métier saura choisir une concentration adéquate pour ledit substrat.

Conformément à l'invention, un moyen d'analyse électrochimique pour la mise en oeuvre de l'invention peut être une mesure potentiométrique, une mesure d'impédancemétrie, une mesure coulométrique ou une mesure ampérométrique.

Dans un mode de réalisation avantageux, l'analyse électrochimique est mise en oeuvre par une mesure ampérométrique.

On entend par « mesure ampérométrique » une mesure du courant électrique en fonction d'une différence de potentiel établie entre l'électrode de travail et l'électrode de référence.

La mesure du courant électrique peut être réalisée au moyen des techniques ampérométriques connues, préférentiellement par une voltampérométrie à balayage de potentiel pouvant être linéaire, cyclique, à impulsion, ou encore du type à saut de potentiel, tel que la chronoampérométrie.

Dans un mode de réalisation particulièrement avantageux de l'invention, la présence d'un substrat des bêta-lactamases hydrolysé est mesurée par la voltampérométrie cyclique ou linéaire.

La mise en oeuvre de ces techniques requière un montage pouvant être à deux voire à trois électrodes, c'est-à-dire un montage comprenant une électrode de travail, une électrode de référence et éventuellement une électrode auxiliaire (contre-électrode). L'électrode de travail, dont la surface sert de site pour le transfert d'électrons, peut être à base de carbone ou à base d'un métal noble ou encore à base d'oxyde métallique. L'électrode de référence est une électrode dont le potentiel est constant, ce qui permet d'imposer un potentiel précisément défini à l'électrode de travail. L'électrode de référence peut être une électrode Ag/AgCl. La contre-électrode, qui permet d'établir le passage du courant électrique avec l'électrode de travail peut être fabriquée avec un matériau inerte, tel que le platine ou le carbone. L'homme du métier saura choisir et combiner les électrodes appropriées selon ses connaissances générales.

Concernant le mode de fabrication des électrodes, la technique de sérigraphie est préférable, bien que d'autres méthodes de fabrication industrielle telles que la rotagravure, l'impression à jet d'encre ou éventuellement la photolithographie puissent être envisagées. Les électrodes obtenues par sérigraphie sont particulièrement bien adaptées car elles peuvent être produites en masse à faible coût, et donc être éventuellement à usage unique. De plus, leur forme géométrique ainsi que leur taille peuvent être aisément modulables. Ces électrodes peuvent être sérigraphiées sous forme d'un capteur et éventuellement intégrées dans le fond des puits d'une microplaque ou d'autres supports ou systèmes permettant la filtration des suspensions bactériennes et l'incubation avec la nitrocéfine ou un autre substrat des bêta-lactamases possédant des propriétés électrochimiques.

Dans un mode de réalisation particulier, la mesure ampérométrique est mise en oeuvre avec un capteur sérigraphié. Il permet d'effectuer la mesure dans un petit volume de solution de l'ordre de quelques microlitres.

Dans un mode de réalisation particulier, la mesure ampérométrique est mise en oeuvre avec un dispositif impliquant trois électrodes : une électrode de référence Ag/AgCl, une électrode de travail en carbone et une contre-électrode en carbone.

Dans un autre mode de réalisation particulier, la mesure ampérométrique est mise en œuvre avec un capteur sérigraphié comprenant une électrode de référence Ag/AgCl, une électrode de travail en carbone et une contre-électrode en carbone.

La présence d'un substrat hydrolysé, par exemple la nitrocéfine hydrolysée, est indiquée par la présence d'un courant d'oxydation anodique dans un intervalle de potentiels et l'absence dudit courant pour un contrôle dépourvu dudit substrat hydrolysé.

Lorsqu'un substrat hydrolysé, notamment une bêta-lactamine hydrolysée, par exemple la nitrocéfine hydrolysée, est soumise à une mesure par voltampérométrie cyclique, sa présence est indiquée par un pic de courant d'oxydation anodique spécifique au substrat hydrolysé dans un intervalle de potentiels déterminé.

Par exemple, lorsque la détection est réalisée par voltampérométrie cyclique à l'aide d'une électrode de référence Ag/AgCl dans un tampon de pH neutre contenant, par exemple, la nitrocéfine, l'intensité liée au courant de pic d'oxydation anodique correspondant à l'oxydation de nitrocéfine hydrolysée est mesurée dans un intervalle de potentiels compris entre + 0,1 V et +0,5 V, notamment entre + 0,2 V et +0,4 V, plus particulièrement entre +0,23V et + 0,33V.

En particulier, lorsque la détection est réalisée par voltampérométrie cyclique dans un tampon phosphate de pH7 contenant la nitrocéfine, le courant de pic d'oxydation anodique lié à l'oxydation de nitrocéfine hydrolysée se situe à + 0,3 V *vs.* Ag/AgCl.

Lorsqu'un autre type d'électrode de référence et/ou un autre milieu réactionnel est utilisé pour mettre en oeuvre la méthode l'invention, l'homme du métier saura calculer ou identifier cet intervalle de potentiels selon ses connaissances générales.

Etant donné que la présence d'un substrat hydrolysé, par exemple la nitrocéfine hydrolysée ou le composé HMRZ-86 hydrolysé, est détectée par une méthode électrochimique, la méthode décrite dans l'invention s'avère particulièrement avantageuse pour l'analyse d'échantillons colorés ou troubles sans traitement préalable, avec la possibilité d'offrir une mesure quantifiée avec bonne sensibilité à l'aide d'un appareillage de faible coût, de taille réduite, et éventuellement portable.

Un échantillon susceptible d'être analysé par la méthode de l'invention peut être un prélèvement biologique, un échantillon d'origine environnementale ou un échantillon alimentaire.

Un prélèvement biologique peut être notamment un échantillon sanguin, un échantillon urinaire, un échantillon tissulaire ou un échantillon pulmonaire.

Un échantillon d'origine environnementale peut être les eaux usées, les eaux usées hospitalières, les eaux usées traitées ou les boues issues du traitement des eaux usées, ainsi que des résidus de réacteurs de méthanisation par voie humide ou sèche (digestats).

Un échantillon alimentaire peut être les produits et sous-produits d'origine animale et plus particulièrement d'origine aviaire comme les volailles, susceptibles de contenir des bactéries résistantes aux bêta-lactamases.

Particulièrement, la méthode de l'invention comprend les étapes suivantes :
(a) incuber un échantillon susceptible de contenir lesdites bactéries dans un milieu contenant un substrat des bêta-lactamases, notamment une bêta-lactamine, possédant des propriétés électrochimiques,
(b) appliquer une mesure ampérométrique respectivement au susdit milieu obtenu à la fin de l'étape (a) et à un contrôle négatif,
(c) déterminer la présence des bactéries produisant des bêta-lactamases par la comparaison de la valeur de l'intensité du courant anodique correspondant à l'oxydation du substrat hydrolysé, notamment de la bêta-lactamine hydrolysée, mesurée pour le susdit milieu obtenu à la fin de l'étape (a), avec la valeur de l'intensité du courant anodique mesurée pour le contrôle négatif.

Conformément à l'invention, un contrôle négatif est un milieu contenant ledit substrat en absence d'échantillon.

Plus particulièrement, la méthode de l'invention comprend les étapes suivantes :
(a) incuber un échantillon susceptible de contenir lesdites bactéries dans un milieu contenant la nitrocéfine,
(b) appliquer une mesure ampérométrique respectivement au susdit milieu obtenu à la fin de l'étape (a) et à un contrôle négatif,
(c) déterminer la présence des bactéries produisant des bêta-lactamases par la comparaison de la valeur de l'intensité du courant anodique correspondant à l'oxydation de la nitrocéfine hydrolysée mesurée pour le susdit milieu obtenu à la fin de l'étape (a), avec la valeur de l'intensité du courant anodique mesurée pour le contrôle négatif.

Une mesure ampérométrique appliquée audit contrôle négatif permet d'indiquer tout courant qui n'est pas lié à l'oxydation de la nitrocéfine hydrolysée.

Lors d'une mesure ampérométrique, la présence de la nitrocéfine hydrolysée est indiquée par l'apparition ou l'augmentation d'un courant anodique dans un intervalle de potentiels déterminé par rapport au courant anodique du contrôle mesuré dans le même intervalle.

Par ailleurs, la méthode décrite dans la présente invention offre la possibilité de quantifier les bactéries produisant des bêta-lactamases dans un échantillon.

En effet, l'intensité du courant anodique produit par l'oxydation d'un substrat hydrolysé, notamment de la nitrocéfine hydrolysée, est quantitativement proportionnelle à la quantité de nitrocéfine hydrolysée. Lorsque la quantité du substrat est en excès, la quantité du substrat hydrolysé est aussi proportionnelle à la quantité des bêta-lactamases impliquées dans l'hydrolyse dudit substrat, ainsi qu'à la quantité des bactéries produisant ces enzymes. Par conséquent, la comparaison de la valeur de l'intensité du courant anodique spécifique au substrat hydrolysé avec une valeur de référence, lue sur une courbe de calibration, permet de déterminer la quantité des bactéries produisant des bêta-lactamases.

Un mode de réalisation particulier de l'invention permet à la fois de déterminer la présence des bactéries produisant des bêta-lactamases dans un échantillon susceptible de les contenir et de les quantifier.

Dans ce mode de réalisation, la méthode comprend en outre, après la détermination de la présence de bactéries productrices de bêta-lactamases, une étape consistant à comparer la valeur de l'intensité du courant anodique mesurée pour ledit milieu obtenu à la fin de l'étape (a) avec une courbe d'étalonnage établie dans les mêmes conditions.

Ladite courbe d'étalonnage est établie en diluant en série un échantillon de référence contenant une quantité connue de bactéries produisant des bêta-lactamases et préalablement déterminée par toute méthode conventionnelle, comme par exemple le dénombrement sur milieu de culture ou encore la PCR en temps réel.

Dans un mode de réalisation particulier, l'invention concerne une méthode permettant la détermination et la quantification des bactéries produisant des bêta-lactamases dans un échantillon susceptible de contenir lesdites bactéries, ladite méthode comprenant :
(a) incuber un échantillon susceptible de contenir lesdites bactéries dans un milieu contenant un substrat des bêta-lactamases, notamment une bêta-lactamine, possédant des propriétés électrochimiques,
(b) appliquer une mesure ampérométrique respectivement au susdit milieu obtenu à la fin de l'étape (a) et à un contrôle négatif,
(c) déterminer la présence des bactéries produisant des bêta-lactamases par comparaison de la valeur de l'intensité du courant anodique correspondant à l'oxydation du substrat hydrolysé, notamment de la bêta-lactamine hydrolysée, mesurée pour le susdit milieu obtenu à la fin de l'étape (a), avec la valeur de l'intensité du courant anodique mesurée pour le contrôle négatif ;
(d) comparer la valeur de l'intensité du courant anodique mesurée pour le susdit milieu obtenu à la fin de l'étape (a) avec une courbe d'étalonnage établie dans les mêmes conditions pour quantifier lesdites bactéries.

Selon la spécificité des substrats possédant des propriétés électrochimiques, la méthode de l'invention peut être mise en oeuvre pour déterminer spécifiquement *in vitro* la présence de bactéries productrices d'un ou plusieurs types des bêta-lactamases.

Dans un mode de réalisation particulier, la méthode de l'invention permet de déterminer et de quantifier *in vitro* la présence de bactéries résistantes à une céphalosporine de 3^{ème} génération, ladite méthode mettant en oeuvre dans l'étape (a) une bêta-lactamine hydrolysée uniquement par les bêta-lactamases à spectre étendu, les céphalosporinases hyperproduites et les carbapénèmases, notamment le composé HMRZ-86 (Isomère E de l'acide (6R,7R)-trifluoroacétate 7-[[2-(2-amino-4-thiazolyl)-2-[(1-carboxy-1-méthyléthoxy)imino]acétyl]amino]-1-aza-3-[2-(2,4-dinitrophényl)éthényl]-8-oxo-5-thiabicyclo[4.2.0]oct-2-ène-2-carboxylique)

Dans un autre mode de réalisation particulier, la méthode de l'invention permet de déterminer et de quantifier *in vitro* la présence de bactéries productrices de carbapénémases, ladite méthode mettant en œuvre dans l'étape (a) un substrat hydrolysé uniquement par les carbapénèmases, notamment le substrat (réactif R2) du kit β CARBA^{™} commercialisé par Bio-rad.

Dans un autre mode de réalisation particulier, à l'aide de la nitrocéfine et d'au moins une autre bêta-lactamine et d'au moins un inhibiteur d'une bêta-lactamase, la méthode de l'invention permet également de discriminer le type des bêta-lactamases produites par les susdites bactéries dans un échantillon susceptible de les contenir.

En effet, les Inventeurs ont constaté que, dans une série de milieux de culture contenant respectivement un substrat spécifique et éventuellement un inhibiteur spécifique à un type de bêta-lactamase, les bactéries productrices des différents types de bêta-lactamases peuvent respectivement donner un profil spécifique en termes d'intensités du courant anodique pour la nitrocéfine hydrolysée lors de la mise en oeuvre d'une mesure ampérométrique.

L'un des avantages de ce mode de réalisation est qu'elle permet la discrimination des bêta-lactamases dans des matrices complexes et colorées sans isolement préalable des bactéries.

Plus particulièrement, une méthode de l'invention permet en outre de discriminer le type de bêta-lactamases choisies parmi les pénicillinases, les bêta-lactamases à spectre étendu, les céphalosporinases inductibles, les céphalosporinases hyperproduites et les carbapénèmases, produites par les susdites bactéries dans un échantillon susceptible de les contenir, ladite méthode comprenant les étapes suivantes :
(a) incuber parallèlement une fraction de susdit échantillon dans un milieu de culture A, un milieu de culture B et un milieu de culture C :
   - le milieu de culture A étant un milieu de culture de base,
   - le milieu de culture B étant un milieu de culture de base complété par une céphalosporine de 3^{ème} génération, et
   - le milieu de culture C étant un milieu de culture de base complété par une céphalosporine et un inhibiteur de pénicillinases, et
   éventuellement dans un milieu de culture B', un milieu de culture C', un milieu de culture D, un milieu de culture E :
   - le milieu de culture B' étant un milieu de culture de base complété par une céphalosporine de 3^{ème} génération différente de celle présente dans le milieu B ;
   - le milieu de culture C' étant le milieu de culture B' complété par un inhibiteur de pénicillinases ;
   - le milieu de culture D étant un milieu de culture de base complété par une céphalosporine et un inhibiteur des céphalosporinases, et
   - le milieu de culture E étant un milieu de culture de base complété par un carbapénème,
(b) incuber parallèlement les susdits milieux de culture obtenus à la fin de l'incubation de l'étape (a) dans un milieu contenant la nitrocéfine;
(c) appliquer un moyen ampérométrique aux susdits milieux obtenus à la fin de l'étape (b) pour déterminer la présence de bactéries produisant des bêta-lactamases et discriminer le type des bêta-lactamases.

Conformément à l'invention, un milieu de culture de base peut être tout type de milieu de culture utilisé conventionnellement pour incuber les bactéries, tel que le milieu LB (Luria Bertani broth), TSB (Tryptone soy broth) ou BHI (Brain heart infusion).

Dans un mode de réalisation particulier, avant la mise en oeuvre de l'étape (b) et après l'étape (a), la susdite méthode de l'invention comprend en outre, une étape pour concentrer les bactéries présentes dans les milieux de culture afin de donner une réponse catalytique plus significative. La concentration des bactéries peut être effectuée, par exemple, par filtration ou centrifugation.

Conformément à l'invention, ladite céphalosporine de 3^{ème} génération est choisie parmi le céfotaxime, la ceftazidime et la ceftriaxone; ledit carbapénème est choisi parmi l'ertapémème, l'imipénème ou le méropénème.

Conformément à l'invention, ledit inhibiteur de pénicillinases est notamment choisi parmi l'acide clavulanique, le tazobactam ou le sulbactam; ledit inhibiteur des céphalosporinases est notamment la cloxacilline.

Le milieu de culture B ou B' permet d'inhiber la croissance des bactéries produisant des pénicillinases ou des céphalosporinases inductibles.

Le milieu de culture C ou C' permet d'inhiber la croissance des bactéries produisant une pénicillinase ou une bêta-lactamase à spectre étendu. Dans un mode de réalisation particulier, un milieu de culture C est un milieu de culture B complété par un inhibiteur de pénicillinases.

Le milieu de culture D permet d'inhiber la croissance des bactéries produisant une céphalosporinase inductible ou hyperproduite.

Le milieu de culture E permet d'inhiber la croissance des bactéries produisant toute bêta-lactamase sauf une carbapénèmase.

L'utilisation des milieux B, B', C et C' permet de discriminer les bactéries productrices de BLSEs qui sont sensibles à une céphalosporine de 3^{ème} génération, par exemple le céfotaxime, mais résistantes à une autre céphalosporine de 3^{ème} génération par exemple la ceftazidime, ou inversement.

Après l'incubation respective des fractions d'un même échantillon dans les milieux de culture A, B, C, et éventuellement les milieux B', C', D et E, les bêta-lactamases produites par les bactéries issues de ces fractions se différencient. La présente invention permet d'indiquer cette différence par une méthode ampérométrique qui indique la quantité de nitrocéfine hydrolysée par ces bêta-lactamases par les valeurs de l'intensité du courant anodique correspondant à l'oxydation de la nitrocéfine hydrolysée.

Les valeurs i_{A}, i_{B}, i_{B}' i_{C}, i_{C}', i_{D}, et i_{E} sont les valeurs de l'intensité du courant anodique correspondant à l'oxydation de la nitrocéfine hydrolysée respectivement mesurées pour les bactéries issues des milieux de culture A, B, B', C, C', D et E.

Dans le cadre de la méthode de l'invention, une pénicillinase est caractérisée par la présence des courants spécifiques à la nitrocéfine hydrolysée obtenus pour les bactéries issues d'un milieu A, d'un milieu B et éventuellement d'un milieu B', et l'absence du courant spécifique pour les bactéries issues d'un milieu C et éventuellement d'un milieu C'. La valeur i_{A} peut être soit inférieure soit supérieure à la valeur i_{B} et éventuellement i_{B'}.

Une BLSE est caractérisée par une forte intensité du courant anodique spécifique à la nitrocéfine hydrolysée obtenue pour les bactéries issues respectivement d'un milieu A, d'un milieu B et éventuellement d'un milieu B' et l'absence de ce courant spécifique pour les bactéries issues d'un milieu C et éventuellement d'un milieu C'. Les valeurs i_{B} et éventuellement i_{B}' sont proches de la valeur i_{A}.

Une céphalosporinase inductible est caractérisée par un courant anodique spécifique à la nitrocéfine hydrolysée obtenue pour les bactéries issues des milieux A, B et éventuellement B', C et éventuellement C', avec i_{A} < i_{B} < i_{C} et éventuellement i_{A} < i_{B'} < i_{C'}, et l'absence de ce courant spécifique pour les bactéries issues d'un milieu D.

Une céphalosporinase hyperproduite est caractérisée par une forte intensité du courant anodique spécifique à la nitrocéfine hydrolysée obtenue pour les bactéries issues d'un milieu A, la présence de ce courant spécifique pour les bactéries issues respectivement d'un milieu B et d'un milieu C, éventuellement d'un milieu B' et d'un milieu C', avec i_{B} > i_{C} et éventuellement i_{B'} > i_{C}, et l'absence de ce courant spécifique pour les bactéries issues d'un milieu D.

Une carbapénèmase est caractérisée par la présence d'un courant anodique spécifique à la nitrocéfine hydrolysée obtenu pour les bactéries issues des milieux A, B et éventuellement B', C et éventuellement C', D et E.

Dans un mode de réalisation plus particulier, l'invention concerne une méthode pour déterminer la présence des bactéries produisant une bêta-lactamase à spectre étendu, ladite méthode comprenant les étapes suivantes:
(a) incuber parallèlement une fraction de susdit échantillon dans un milieu de culture A, un milieu de culture B et un milieu de culture C tels que définis auparavant,
(b) incuber parallèlement les susdits milieux de culture obtenus à la fin de de l'incubation de l'étape (a) dans un milieu contenant la nitrocéfine,
(c) appliquer un moyen ampérométrique aux susdits milieux obtenus à la fin de l'étape (b) pour déterminer la présence des bactéries produisant une bêta-lactamase à spectre étendu.

Dans un autre mode de réalisation plus particulier, l'invention concerne une méthode pour déterminer la présence des bactéries produisant une bêta-lactamase à spectre étendu, ladite méthode comprenant les étapes suivantes:
(a) incuber parallèlement une fraction du susdit échantillon dans un milieu de culture A, un milieu de culture B, un milieu de culture B', un milieu de culture C et un milieu de culture C', tels que définis auparavant,
(b) incuber parallèlement les susdits milieux de culture obtenus à la fin de de l'incubation de l'étape (a) dans un milieu contenant la nitrocéfine,
(c) appliquer un moyen ampérométrique aux susdits milieux obtenus à la fin de l'étape (b) pour déterminer la présence des bactéries produisant une bêta-lactamase à spectre étendu.

L'utilisation des milieux B, B', C et C' permet de discriminer les bactéries productrices de BLSEs qui sont sensibles à une céphalosporine de 3^{ème} génération mais résistantes à une autre céphalosporine de 3^{ème} génération.

Dans un mode de réalisation particulier, le milieu de culture B est le milieu de culture A complété par le céfotaxime ; le milieu de culture B' est le milieu de culture A complété par la ceftazidime ; le milieu de culture C est le milieu de culture B complété par un inhibiteur de pénicillinase; le milieu de culture C' est le milieu de culture B' complété par un inhibiteur de pénicillinases.

Dans un autre mode de réalisation particulier, le milieu de culture B est le milieu de culture A complété par la ceftazidime ; le milieu de culture B' est le milieu de culture A complété par le céfotaxime ; le milieu de culture C est le milieu de culture B complété par un inhibiteur de pénicillinases ; le milieu de culture C' est le milieu de culture B' complété par un inhibiteur de pénicillinases.

L'utilisation de ces milieux dans le cadre de l'invention permet de discriminer les bactéries productrices de BLSEs qui sont soit sensibles au céfotaxime mais résistantes à la ceftazidime ou soit résistantes au céfotaxime mais sensibles à la ceftazidime.

Avantageusement, ladite méthode pour déterminer la présence de bactéries productrices des bêta-lactamases et discriminer le type des bêta-lactamases comprend les étapes suivantes :
(a) incuber parallèlement une fraction de susdit échantillon dans un milieu de culture A, un milieu de culture B, un milieu de culture B', un milieu de culture C et un milieu de culture C':
   - le milieu de culture A étant un milieu de culture de base,
   - le milieu de culture B et le milieu de culture B' étant des milieux de culture de base complétés respectivement par une céphalosporine de 3^{ème} génération différente,
   - le milieu de culture C et le milieu de culture C' étant respectivement les milieux de culture B et B' complétés par un inhibiteur de pénicillinases,
   et éventuellement dans un milieu de culture D, un milieu de culture E:
   - le milieu de culture D étant un milieu de culture de base complété par une céphalosporine et un inhibiteur de céphalosporinases, et
   - le milieu de culture E étant un milieu de culture de base complété par un carbapénème,
(b) incuber parallèlement les susdits milieux de culture obtenus à la fin de l'incubation de l'étape (a) dans un milieu contenant la nitrocéfine;
(c) appliquer un moyen ampérométrique aux susdits milieux obtenus à la fin de l'étape (b) pour déterminer la présence de bactéries produisant des bêta-lactamases et discriminer le type des bêta-lactamases.

Selon un autre mode de réalisation plus particulier, la méthode de l'invention permet de déterminer et de discriminer les bactéries produisant des bêta-lactamases dans un échantillon susceptible de les contenir, ladite méthode comprenant les étapes suivantes :
(a) incuber parallèlement une fraction du susdit échantillon dans un milieu de culture A, un milieu de culture B, un milieu de culture C, un milieu de culture D, un milieu de culture E, éventuellement un milieu B' et un milieu C', tels que définis auparavant,
(b) incuber parallèlement les susdits milieux de culture obtenus à la fin de l'incubation de l'étape (a) dans un milieu contenant la nitrocéfine,
(c) appliquer une mesure ampérométrique respectivement aux susdits milieux obtenus à la fin de l'étape (b) et à un contrôle négatif,
(d) déterminer la présence de bactéries produisant des bêta-lactamases dans ledit échantillon par la comparaison de la valeur de l'intensité du courant anodique correspondant à l'oxydation de nitrocéfine hydrolysée obtenu pour la fraction cultivée dans le milieu de culture A avec la valeur de l'intensité du courant obtenu pour le contrôle négatif, et
(e) discriminer le type de bêta-lactamases produites par les susdites bactéries dans ledit échantillon, par la comparaison des valeurs respectives de l'intensité du susdit courant anodique obtenues pour les fractions cultivées parallèlement dans les milieux de culture A, B, C, D et E, éventuellement B' et C' avec les valeurs respectives obtenues pour une souche bactérienne de référence.

On entend par "souche bactérienne de référence" une souche bactérienne produisant une bêta-lactamase dont le type est déjà identifié.

Dans un mode de réalisation encore plus particulier, la méthode de l'invention comprend en outre une étape (f) après l'étape (e) permettant de quantifier des bactéries produisant la bêta-lactamase discriminée dans l'étape (e) par comparaison de la valeur de l'intensité du courant anodique correspondant à la nitrocéfine hydrolysée par ladite bêta-lactamase avec une courbe d'étalonnage établie dans les mêmes conditions.

La valeur de l'intensité du courant anodique correspondant à la nitrocéfine hydrolysée par une bêta-lactamase à spectre étendu est calculée selon la formule: i_{B}-i_{C}.

La valeur de l'intensité du courant anodique correspondant à la nitrocéfine hydrolysée par une céphalosporinase hyperproduite est calculée selon la formule: i_{B}-i_{D}.

La valeur de l'intensité du courant anodique correspondant à la nitrocéfine hydrolysée par une carbapénèmase correspond à la valeur i_{E}.

Plus avantageusement, la méthode de l'invention peut même discriminer les bactéries produisant une sous-famille de bêta-lactamase, à l'aide d'un milieu supplémentaire contenant un substrat et un inhibiteur spécifique à une sous-famille d'une bêta-lactamase.

La méthode de l'invention peut notamment discriminer les bactéries produisant une sous-famille de carbapénèmase, en utilisant un milieu supplémentaire contenant un carbapénème et un inhibiteur spécifique à une sous famille de carbapénèmases.

La discrimination est basée sur la sensibilité spécifique d'une sous-famille de carbapénèmase à certains types d'inhibiteurs. Par exemple, les métallo-bêta lactamases, une sous-famille de carbapénèmases appartenant à la classe B selon Ambler, sont sensibles à l'EDTA ou à l'acide mercaptoacétique, alors que les carbapénèmases de classe A selon la classification Ambler sont sensibles à l'acide clavulanique et à l'acide boronique.

Conformément à l'invention, un inhibiteur de carbapénèmases est choisi parmi l'EDTA, l'acide mercaptoacétique, l'acide boronique, ou l'acide clavulanique.

Dans un autre mode de réalisation avantageux, la méthode de l'invention permet de discriminer le type des bêta-lactamases et éventuellement de préciser la sous-famille de carbapénèmases produites par les susdites bactéries dans un échantillon susceptible de les contenir, ladite méthode comprenant les étapes suivantes :
(a) incuber parallèlement une fraction du susdit échantillon :
   - dans un milieu de culture A, un milieu de culture B et un milieu de culture C, tels que définis auparavant, et
   - éventuellement dans un milieu de culture B', un milieu de culture C', un milieu de culture D, un milieu de culture E, et un milieu de F, les milieux de culture B', C', D, E étant tels que définis auparavant, le milieu de culture F étant un milieu de culture de base complété par un carbapénème et un inhibiteur spécifique à une sous-famille de carbapénèmases;
(b) incuber parallèlement les susdits milieux de culture obtenus à la fin de l'étape (a) dans un milieu contenant la nitrocéfine,
(c) appliquer un moyen d'analyse électrochimique aux susdits milieux obtenus à la fin de l'étape (b) pour déterminer la présence de bactéries produisant des bêta-lactamases et discriminer le type des bêta-lactamases.

Le milieu de culture F permet d'inhiber la croissance des bactéries produisant un type de carbapénèmase qui est sensible au susdit inhibiteur spécifique à une sous-famille de carbapénèmases : l'EDTA et l'acide mercaptoacétique sont des inhibiteurs spécifiques de la classe B alors que l'acide boronique et l'acide clavulanique sont des inhibiteurs spécifiques de la classe A.

Dans un mode de réalisation particulièrement avantageux, la méthode de l'invention pour discriminer le type des bêta-lactamases et éventuellement préciser la sous-famille de carbapénèmases produites par les susdites bactéries comprend les étapes suivantes :
(a) incuber parallèlement une fraction du susdit échantillon dans un milieu de culture A, un milieu de culture B, un milieu de culture C, un milieu de culture D, un milieu de culture E, et un milieu de culture F, tels que définis auparavant, et éventuellement un milieu de culture B' et un milieu de culture C' tels que définis auparavant,
(b) incuber parallèlement les susdits milieux de culture obtenus à la fin de l'incubation de l'étape (a) dans un milieu contenant la nitrocéfine,
(c) appliquer une mesure ampérométrique respectivement aux susdits milieux obtenus à la fin de l'étape (b) et à un contrôle négatif,
(d) déterminer la présence de bactéries produisant des bêta-lactamases dans ledit échantillon par la comparaison de la valeur de l'intensité du courant anodique correspondant à l'oxydation de nitrocéfine hydrolysée obtenue pour la fraction cultivée dans le milieu de culture A avec la valeur de l'intensité du courant obtenue pour le contrôle négatif,
(e) discriminer le type de bêta-lactamases produites par les susdites bactéries dans ledit échantillon, par la comparaison des valeurs respectives de l'intensité du susdit courant anodique obtenues pour les fractions cultivées parallèlement dans les milieux de culture A, B, C, D, E et F, et éventuellement les milieux de culture B' et C', avec les valeurs respectives obtenues pour une souche bactérienne de référence.

La méthode de la présente invention peut être utilisée au cours du développement de nouveaux antibiotiques de la famille des β-lactamines en particulier pour l'étude de la stabilité de la molécule vis-à-vis de différentes bêta-lactamases.

La présente invention est illustrée plus en détail par les figures et les exemples ci-après.

### Figures

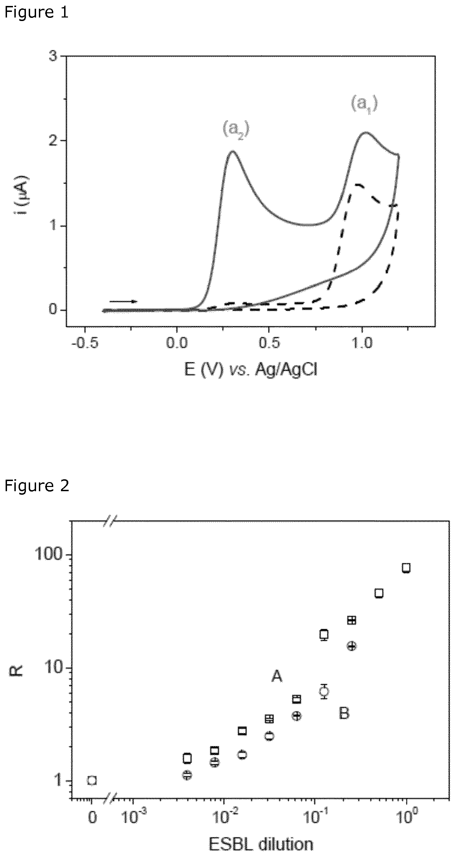

Figure 1 : Voltammogrammes cycliques (v = 50 mV.s⁻¹) obtenus pour une solution de nitrocéfine à 500 µM dans du PBS en absence (trait pointillé) et en présence de BLSE (trait plein) après 10 min d'incubation.
Figure 2 : Courbe d'étalonnage (log-log) d'une BLSE dont les quantités sont déterminées par la méthode de voltampérométrie cyclique selon l'invention (A, □) et par la méthode spectrophotométrique (B, O). Les réponses analytiques sont enregistrées après 10 min d'incubation des BLSEs avec une solution de nitrocéfine à 500 µM préparée dans du PBS. Les milieux réactionnels sont dilués 5 fois dans PBS avant d'effectuer la mesure spectrophotométrique. La valeur R correspond aux réponses de courant ou aux densités optiques, respectivement normalisées avec les valeurs obtenues en absence de BLSE (i₀ = 50 ± 5 nA; OD₀ = 0,097 ± 0,008). L'écart type représente l'erreur standard des deux mesures.

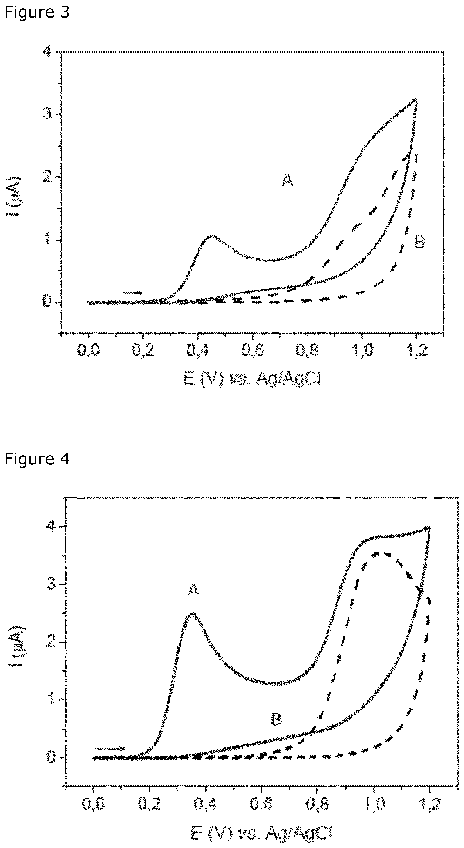

Figure 3: Courbes voltammétriques (v = 50 mV.s⁻¹) enregistrées sur un capteur sérigraphié pour les souches *E. coli* BLSE⁺ (A) et BLSE⁻ (B), cultivées à 37°C pendant 4h30 dans un milieu de culture contenant 4 µg.mL⁻¹ de céfotaxime après la centrifugation (1 mL; 7000 g; 10 min) et l'incubation du culot pendant 10 min avec 50 µL d'une solution de nitrocéfine à 500 µM dans du PBS.
Figure 4 : Voltammogrammes cycliques (v = 50 mV.s⁻¹) enregistrés avec un capteur sérigraphié pour la souche *E. coli* BLSE⁺ cultivée dans un milieu de culture contenant uniquement 4 µg.mL⁻¹ de céfotaxime (A) ou complété par 10 µg.mL⁻¹ de clavulanate de potassium, puis centrifugée (1 mL; 7000 g; 10 min) avant une étape d'incubation du culot avec 50 µL dans une solution de nitrocéfine à 500 µM dans du PBS pendant 10 min.

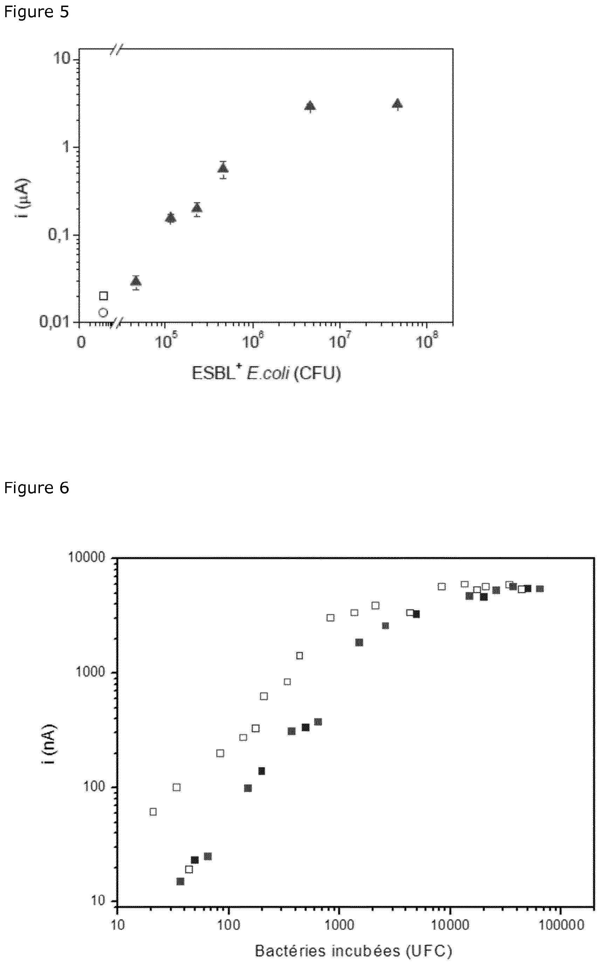

Figure 5: Courbe de calibration de la souche BLSE⁺ après l'incubation à 37°C pendant 3h30 dans un milieu de culture LB complété par 4 µg.mL⁻¹ de céfotaxime. La culture bactérienne est ensuite diluée en série et des volumes de de 10 mL sont filtrés en double. Les bactéries récupérées après la filtration sont incubées pendant 10 min avec 80 µL de la solution de nitrocéfine à 500 µM dans PBS. L'axe des abscisses correspond au nombre de bactéries récupérées après la filtration. Les barres d'erreur représentent la déviation standard de deux expériences. Le carré ouvert (□) représente le contrôle négatif consistant en la souche BLSE⁺ cultivée avec 4 µg.mL⁻¹ de céfotaxime et 10 µg.mL⁻¹ d'acide clavulanique. Le cercle ouvert (O) correspond au signal de la nitrocéfine non hydrolysée.
Figure 6 : Courbes d'étalonnage d'une souche de E. *coli* produisant des BLSEs réalisées dans trois échantillons d'eaux usées brutes (■) et trois échantillons d'eaux traitées préalablement autoclavées (□). La valeur i (nA) correspond au courant du pic d'oxydation de la nitrocéfine hydrolysée enregistré pour une quantité de bactéries auquel a été retranché celui mesuré pour le témoin négatif.

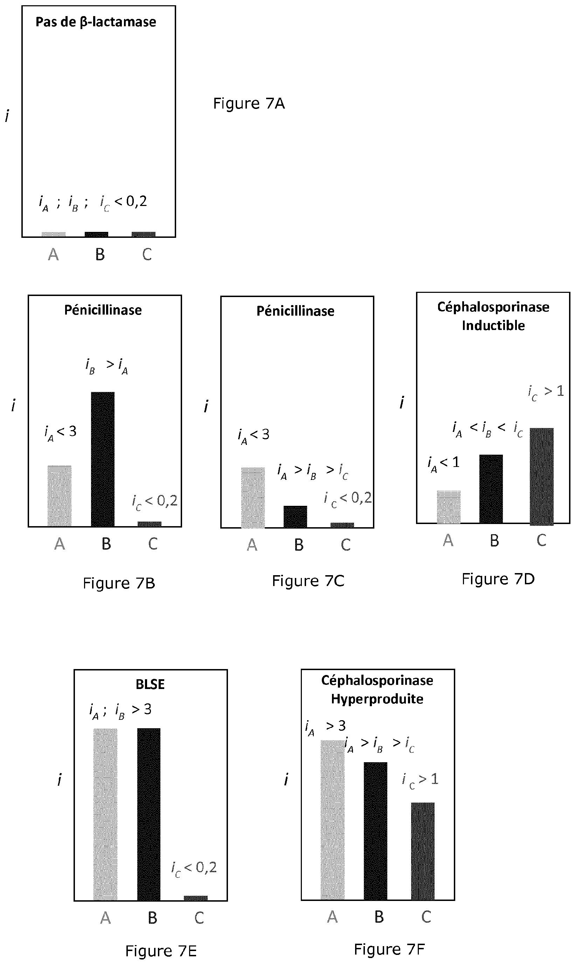

Figures 7A, 7B, 7C, 7D, 7E, 7F: Profils des courants d'oxydation (µA) de la nitrocéfine hydrolysée enregistrés pour différents types de bêta-lactamases et utilisés pour discriminer les bêta-lactamases dans des échantillons médicaux (hémocultures, souches isolées).

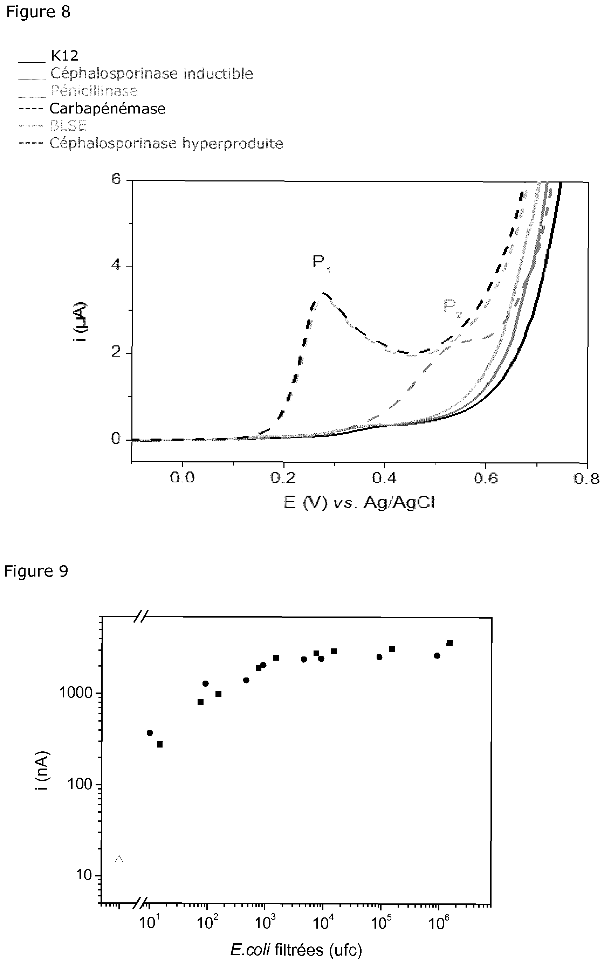

Figure 8 : Voltammogrammes cycliques (v = 50 mV.s⁻¹) enregistrés pour six cultures liquides (v=50 µL) contenant respectivement une souche non productrice de bêta-lactamase (K12), une souche productrice de céphalosporinase inductible, une souche productrice de pénicillinase, une souche productrice de BLSE, une souche productrice de céphalosporinase hyperproduite, une souche productrice de carbapénémase après une incubation avec 50 µL de HMRZ-86 (0,5 mM dans PBS) pendant 30 min.
Figure 9 : Courbes d'étalonnages réalisées avec deux souches d'E. *coli* productrices de BLSE (type CTX-M15) représentées respectivement par les symboles ▪ et • dans un échantillon d'eau usée autoclavée. La valeur i (nA) correspond au courant de pic d'oxydation du HMRZ-86 auquel a été retranché celui mesuré pour le témoin négatif avec une valeur moyenne de 235 ± 15 nA (Δ).

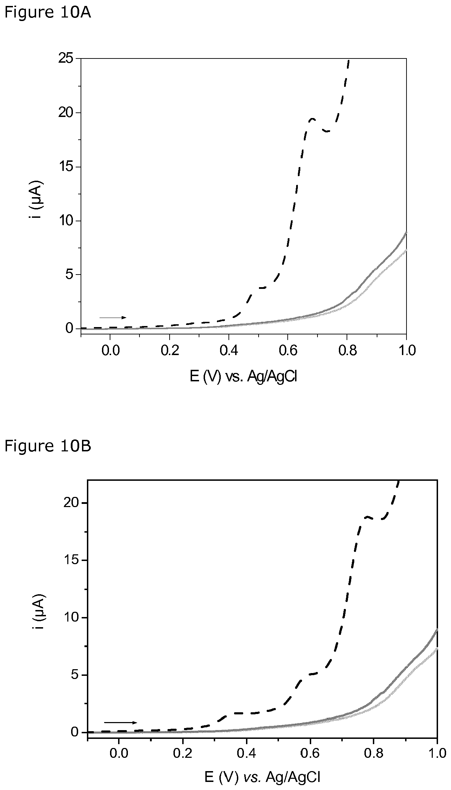

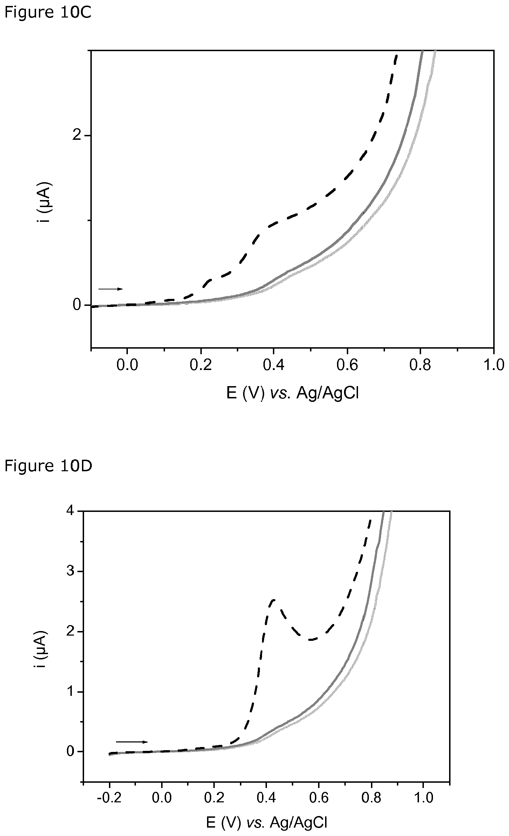

Figures 10A, 10B, 10C, 10D : Chaque figure représente les voltammogrammes cycliques (v = 50 mV.s⁻¹) enregistrés pour trois solutions de de Carba-S (30 µL) incubées à 37°C pendant 30 min respectivement avec une souche productrice de BLSE, une souche productrice de céphalosporinases hyperproduites et une souche productrice de carbapénémases. Figure 10A : la souche productrice de carbapénémase OXA-48. Figure 10B : la souche productrice de carbapénémase de type OXA-48 + CTX-M. Figure 10C : la souche productrice de carbapénémase de type NDM-1. Figure 10D : la souche productrice de carbapénémase de type KPC-2.

### Exemples

### Exemple I : Identification des bactéries produisant des bêta-lactamases utilisant la nitrocéfine comme substrat

### 1. Matériels et méthodes

### 1.1. Réactifs et solutions

Le tampon phosphate (PBS ; 100 mM; pH = 7,0) et toutes les solutions aqueuses sont préparées avec de l'eau Milli-Q 18-MΩ (Millipore purification system).

Le milieu de culture, composé de bouillon Luria, (LB, 10 g.L⁻¹ de Bacto tryptone, 5 g.L⁻¹ d'extrait de levure Bacto, 5 g.L⁻¹ de NaCl) est préparé au laboratoire et stérilisé par autoclavage à 120 °C.

La nitrocéfine est achetée chez Merck Millipore (Référence 484400). Une solution mère de nitrocéfine à 10⁻² M est préparée dans du diméthylsulfoxyde (DMSO) puis stockée sous forme d'aliquotes de 50 µL à - 20°C.

Le sel sodique de céfotaxime (Référence C 7039-100mg) et le clavulanate de potassium (Référence 33454-100 mg) sont fournis par Sigma-Aldrich. Des solutions mères à 1 mg.mL⁻¹ sont préparées quotidiennement dans du PBS.

La BLSE a été extraite d'une souche de *E. coli* isolée au laboratoire de Bactériologie du Centre Hospitalier Universitaire de Dijon (1485 ; type CTX-M-1).

La souche E. *coli* K12 et la souche E. *coli* productrice de BLSE (MIAE6690) proviennent de la collection de souches de l'INRA Dijon. Des échantillons de ces souches sont stockés à -80 °C sous forme d'aliquote de 500 µL contenant 50 % de suspension bactérienne et 50 % de glycérol (v/v).

### 1.2. Appareils de mesure et électrodes

### 1.2.1. Pour la preuve de concept

Les mesures électrochimiques sont réalisées avec un potentiostat PGSTAT12 Autolab (Metrohm) piloté par le logiciel GPES (version 4.9).

Les capteurs sont préparés avec une machine à sérigraphier manuelle (Circuit Imprimé Français, Bagneux, France) avec de l'encre de carbone (Electrodag ^{®} PF 407A, Acheson Colloids). Une série de 6 capteurs est imprimée sur un film souple de polyester en faisant passer de l'encre à travers un cadre de sérigraphie (120 fils/cm) à l'aide d'une raclette. Après une étape de séchage (1heure à 65°C), les capteurs sont ensuite stockés à température ambiante.

Chaque capteur est constitué de l'électrode de travail et d'une contre-électrode. La surface de travail du capteur (7,07 mm²) est délimitée avec un anneau adhésif qui permet également de définir la cellule électrochimique. Pour effectuer la mesure ampérométrique, le capteur est inséré dans un connecteur (Dropsens) relié au potentiostat puis une goutte de 20 µL de la solution à analyser est déposée sur la surface du capteur. Après avoir plongé un fil d'argent recouvert avec un précipité de chlorure d'argent (Ag/AgCl) qui constitue l'électrode de référence, les mesures sont réalisées par voltammétrie cyclique (v = 50 mV.s⁻¹) à température ambiante.

### 1.2.2 Pour la discrimination des bêta-lactamases dans les échantillons sanguins et la quantification des souches productrices de BLSE :

Les mesures voltammétriques (v = 50 mV.s⁻¹) sont réalisées en déposant des gouttes de 30 µL de solution sur des capteurs sérigraphiés en carbone fournis par Dropsens (DRP-110) préalablement connectés à un potentiostat portable PSTAT mini 910 (Methrohm) alimenté par la connection USB de l'ordinateur et piloté par le logiciel PSTAT (version 1.0).

### 1.3. Détection ampérométrique de l'activité bêta-lactamase

5 µL d'une solution de BLSE sont introduits dans tube en polypropylène contenant 45 µL d'une solution de nitrocéfine à 0,5 mM dans un tampon phosphate (PBS ; 100 mM; pH = 7,0). Après une étape d'incubation de 10 minutes à température ambiante dans l'obscurité, 20 µL du mélange sont prélevés et transférés sur la surface du capteur de carbone sérigraphié. Une électrode de référence (Ag/AgCl) est alors immergée dans les 20 µL solution précédemment déposés et les mesures de voltammétrie cyclique sont effectuées en procédant de la manière suivante : balayage de potentiel entre -0,4 V et 1,2 V à une vitesse de 50 mV.s⁻¹.

Le pic d'oxydation apparaissant vers +0,3 V résulte de l'hydrolyse du cycle bêta-lactame de la nitrocéfine et peut être choisi comme réponse analytique pour identifier des bêta-lactamases dans un échantillon.

### 1.4. Mesure ampérométrique et colorimétrique de l'activité BLSE avec la nitrocéfine

45 µL d'une solution de nitrocéfine (0,5 mM dans PBS) sont introduits dans un tube en polypropylène contenant 5 µL de la solution de BLSE préalablement diluée dans du PBS. Le mélange réactionnel est incubé pendant 10 min à température ambiante dans l'obscurité. Le produit de la réaction enzymatique est alors détecté par ampérométrie et spectrophotométrie. La mesure électrochimique est réalisée en transférant 20 µL du mélange sur la surface du capteur de carbone sérigraphié afin de réaliser la mesure voltammétrique comme indiqué précédemment. L'intensité du courant de pic d'oxydation mesuré vers + 0,3 V *vs.* Ag/AgCl est choisie comme réponse analytique. Pour la détection spectrophotométrique, la solution est diluée dans PBS (facteur 5) puis pipetée dans des cuves à usage unique (ratiolab^{®} Q-VETTES semi-micro, n°2712120) avant d'effectuer une mesure d'absorbance à λ = 520 nm avec un spectrophotomètre DU ^{®} 800 (Beckman Coulter).

### 1.5. Détection d'une souche productrice de bêta-lactamases

10 µL de la souche *E*. *coli* K12 (BLSE⁻) non productrice de bêta-lactamases et 10 µL de la souche *E.coli* MIAE6690 productrice de BLSE (BLSE⁺) sont parallèlement mis en culture dans un tube contenant 10 mL de milieu LB supplémenté avec du céfotaxime (4 µg.mL⁻¹) pendant 4h30 à 37°C. Ensuite, 1 mL de chaque suspension bactérienne est transféré dans un tube en polypropylène et centrifugé à 7000 g pendant 10 min. Après avoir retiré le surnageant, un volume de 50 µL d'une solution de nitrocéfine (0,5 mM dans PBS) est introduit dans le tube et incubé avec le culot bactérien pendant 10 min à température ambiante dans l'obscurité. Le liquide est alors aspiré et transféré sur le capteur sérigraphié afin de réaliser la mesure voltammétrique comme indiqué dans la partie 1.3.

### 1.6. Identification des souches résistantes aux céphalosporines de 3^{e} génération dans les hémocultures

Les hémocultures fournies par le laboratoire de Bactériologie du CHRU de Dijon sont des prélèvements sanguins qui ont été mis en culture soit dans des flacons Bactec^{™} adaptés pour la recherche de microorganismes principalement aérobies, soit dans des flacons Bactec^{™} dédiés à la croissance des bactéries principalement anaérobies.

Des volumes de 10 µL d'échantillon sont parallèlement introduits dans des tubes contenant 10 mL de milieu LB (Milieu A), 10 mL de milieu LB supplémenté avec du céfotaxime à 4 µg.mL⁻¹ (Milieu B) et 10 mL de milieu LB supplémenté avec du céfotaxime à 4 µg.mL⁻¹ et de l'acide clavulanique à 100 µg.mL⁻¹ (Milieu C). Après une étape d'incubation à 37°C sous agitation pendant 2 heures, 5 mL du contenu de chaque tube est prélevé avec une seringue puis filtré à l'aide d'un dispositif de filtration manuel (Swinnex^{®}, 13 mm, Millipore) équipé d'une membrane de porosité 0,45 µm (HVLP, 13 mm, Millipore). Chaque filtre est ensuite placé au fond d'un micropuits en polystyrène (plaque de 24 puits, Cellstar^{®}, 662160) dans lequel sont alors introduits 80 µL d'une solution de nitrocéfine (0,5 mM dans du PBS), puis laissé à incuber pendant 10 min à température ambiante dans l'obscurité.

40 µL de chaque mélange sont prélevés et transférés sur la surface d'un capteur de carbone sérigraphié (Dropsens, DRP-110) préalablement connecté à un potentiostat portable PSTAT mini 910 (Methrohm) alimenté par la connexion USB de l'ordinateur et piloté par le logiciel PSTAT (version 1.0). Les mesures de voltammétrie linéaire sont réalisées en effectuant un balayage linéaire de potentiel à partir de -0,1 V jusqu'à 1,2 V à une vitesse de 50 mV.s⁻¹.

Le courant de pic (*i*) apparaissant vers + 0,3 V, lié à l'hydrolyse du cycle bêta-lactame de la nitrocéfine, est choisi comme réponse analytique. La comparaison des valeurs de courant i mesurées pour chaque condition d'incubation de l'échantillon (Milieu A, B, C) permet de conclure sur la capacité de la souche à produire ou non une enzyme hydrolysant le céfotaxime.

### 1.7. Quantification des souches productrices de bétâ-lactamases à spectre élargi (BLSE) dans les eaux usées

### 1.7.1. Réalisation d'une courbe d'étalonnage en E. coli productrices de BLSE dans des eaux de stations d'épuration

10 µL de la souche *E.coli* MIAE6690 productrice de BLSE sont mis en culture dans un tube contenant 10 mL de milieu de culture LB supplémenté avec du céfotaxime (4 µg.mL⁻¹) pendant la nuit à 42°C et sous agitation. La concentration en bactéries dans la culture ainsi obtenue (S₀) est déterminée en effectuant des dilutions en série dans un milieu TS contenant de la Tryptone (10 g.L⁻¹) et du NaCl (5g.L⁻¹) puis en étalant les solutions sur des boîtes contenant un milieu gélosé (35,6 g.L⁻¹ Tryptone Bile X-glucose, 4 mg.L⁻¹ céfotaxime).

La culture So est diluée en série (facteurs de dilution compris entre 10 et 10⁸) dans un échantillon d'eau de station d'épuration (soit brute, soit traitée) préalablement autoclavé en préparant des volumes de solution de 1 mL dans des tubes en polypropylène.

Ensuite, 500 µL de ces solutions précédemment diluées (facteurs de dilution compris entre 10⁴ et 10⁸) sont introduits séparément dans un tube contenant 25 mL de milieu LB supplémenté avec du céfotaxime à 4 µg.mL⁻¹ et incubés à 42°C sous agitation pendant 4 heures. Un volume de 500 µL de l'échantillon d'eau de station d'épuration (soit brute, soit traitée) préalablement autoclavé, utilisé pour faire les dilutions de la souche bactérienne, est incubé dans les mêmes conditions (contrôle négatif). Des volumes de 10 mL sont alors prélevés en double dans chaque tube avec une seringue puis filtrés séparément à l'aide d'un dispositif de filtration manuel (Swinnex^{®}, 13 mm, Millipore) équipé d'une membrane de porosité 0,45 µm (HVLP, 13 mm, Millipore). Chaque filtre est ensuite placé au fond d'un micropuits en polystyrène (plaque de 24 puits, Cellstar^{®}, Référence : 662160) dans lequel sont alors introduits 80 µL d'une solution de nitrocéfine (0,5 mM dans PBS), puis laissé à incuber pendant 15 min à température ambiante dans l'obscurité.

40 µL du contenu de chaque puits sont prélevés et transférés sur la surface d'un capteur de carbone sérigraphié (Dropsens, DRP-110) préalablement connectés à un potentiostat portable PSTAT mini 910 (Methrohm) et les mesures de voltammétrie linéaire sont réalisées comme mentionné dans la partie 1.6.

### 1.7.2. Détection des souches productrices de BLSE dans des échantillons d'eaux de stations d'épuration

Des volumes d'eau usée (1 - 10 mL) et d'eau traitée (20 - 100 mL) sont filtrés en double sur des membranes de porosité 0,45 µm (HAWP, 47 mm, Millipore) à l'aide d'une rampe de filtration en inox (Sartorius Combisart). Pour chaque échantillon, une des membranes est introduite dans un tube contenant 25 mL de milieu LB supplémenté avec du céfotaxime à 4 µg.mL⁻¹ (Milieu B) tandis que la seconde est plongée dans un tube contenant 25 mL de milieu LB supplémenté avec du céfotaxime à 4 µg.mL⁻¹ et de l'acide clavulanique à 10 µg.mL⁻¹ (Milieu C). Tous les tubes sont incubés à 42°C sous agitation pendant 4 heures puis leur contenu est filtré et analysé en présence de nitrocéfine comme indiqué en 1.7.1.

Pour un échantillon donné, les intensités des courants de pic mesurés vers 0,3 V pour les incubations dans les milieux B et C sont respectivement notées *i_{B}* et *i_{C}*. La quantité de souches productrices de BLSE est calculée à partir de la valeur de courant *i* = *i_{B}* ― *i_{C}* et à l'aide d'une courbe d'étalonnage (1.7.1).

### 2. Résultats

### 2.1. Caractérisation électrochimique de la nitrocéfine hydrolysée

La nitrocéfine a été hydrolysée par une bêta-lactamase à spectre étendu en quantité abondante. Les comportements voltammétriques de la nitrocéfine (S) et sa forme hydrolysée (P) ont été étudiés par voltammétrie cyclique à l'aide d' électrodes sérigraphiées à base de carbone. La nitrocéfine et la nitrocéfine hydrolysée présentent toutes les deux un pic d'oxydation anodique irréversible (a1) à environ +1,0 V *vs.* Ag/AgCl. Ce pic peut être attribué à l'oxydation du groupe thiol dans le groupe dihydrothiazine ou à d'autres fonctions spécifiques aux dérivés des céphalosporines. Contrairement à la nitrocéfine, la nitrocéfine hydrolysée (P) génère un pic d'oxydation anodique irréversible (a2) bien défini et observé à un potentiel anodique plus bas d'environ +0,3 V (Fig.1). La présence du pic a2 indique celle de la nitrocéfine hydrolysée et peut donc être choisie comme réponse analytique pour mettre en évidence l'activité de la bêta-lactamase à spectre étendu.

### 2.2. Mesure de l'activité des BLSEs

L'activité des BLSEs a été mesurée parallèlement par une mesure spectrophotométrique et par une mesure voltampérométrique à l'aide de la nitrocéfine. Chaque série d'expériences est effectuée en faisant varier la concentration en BLSEs et les courbes d'étalonnage (log-log) obtenues avec la méthode électrochimique de l'invention et la méthode spectrophotométrique sont illustrées dans la figure 2. Chaque mesure est normalisée par rapport au signal obtenu en absence de BLSE. La courbe d'étalonnage obtenue par la méthode voltampérométrique montre que cette méthode permet une détection quantitative de l'activité de BLSEs avec une sensibilité proche de celle de la méthode spectrophotométrique. De plus, la méthode voltampérométrique de l'invention offre une zone de linéarité plus large. La reproductibilité de la mesure spectrophotométrique et celle de la mesure voltampérométrique sont similaires.

### 2.3. Détection des souches E. coli produisant des BLSEs par mesure de voltampérométrie cyclique

Afin d'évaluer la capacité de la méthode voltampérométrique pour détecter les bactéries produisant des BLSEs, deux souches de *E. coli* dont le génotype est bien caractérisé ont été sélectionnées, l'une étant productrice d'une BLSE (BLSE⁺) et l'autre ne produisant pas de BLSE (BLSE⁻). Les deux souches sont d'abord cultivées dans un milieu LB contenant 4 µg.mL⁻¹ de céfotaxime. Après l'étape de centrifugation, les culots bactériens respectifs de ces deux souches sont incubés avec la nitrocéfine comme substrat. La figure 3 montre les réponses voltammétriques respectivement obtenues pour la souche BLSE⁺ (courbe A) et la souche BLSE⁻ (courbe B). La courbe A présente un pic anodique à environ +0,4 V *vs.* Ag/AgCl, associé à l'hydrolyse catalytique du noyau β-lactame de la nitrocéfine par la souche BLSE⁺, alors qu'aucun pic n'est enregistré pour la souche BLSE⁻. Ce résultat concorde bien avec les valeurs de densité optique de 0,934 et de 0,002 obtenues respectivement pour la souche BLSE⁺ et la souche BLSE⁻. Le milieu de culture complété par le céfotaxime et le milieu sans antibiotique ont également été soumis à la mesure voltampérométrique. Aucun signal spécifique n'est observé dans l'intervalle de potentiels [0,1-0,6 V *vs.* Ag/AgCl] pour ces deux milieux, ce qui confirme que ni le milieu de culture LB, ni le céfotaxime n'interfère avec la détection voltampérométrique.

### 2.4. Discrimination de bactéries produisant des BLSEs

Etant donné que les céphalosporinases hyperproduites et les carbapénèmases peuvent également hydrolyser le céfotaxime, les bactéries produisant ces enzymes peuvent aussi donner une réponse positive en présence de céfotaxime. Afin de distinguer les bactéries produisant les bêta-lactamases à spectre étendu et les bactéries produisant les céphalosporinases hyperproduites et les carbapénèmases, les bactéries ont été cultivées simultanément dans deux milieux de culture, l'un contenant le céfotaxime comme antibiotique, l'autre contenant en plus le clavulanate de potassium comme inhibiteur de BLSEs. Après la récupération des bactéries, les culots bactériens ont été incubés avec la nitrocéfine comme substrat. Les courbes voltammétriques enregistrées par voltampérométrie cyclique sont présentées sur la figure 4. Le pic anodique résultant de l'hydrolyse de la nitrocéfine a été observé pour une souche BLSE⁺ cultivée avec le céfotaxime (courbe A), alors qu'aucun signal n'a été enregistré pour la même souche cultivée en présence du clavulanate de potassium (courbe B). Ce résultat confirme la présence de bactéries productrices de BLSEs.

Ce résultat est en accord avec les valeurs obtenues pour le dénombrement de *E. coli* sur milieu sélectif puisque celles-ci sont respectivement de 80 cfu.mL⁻¹ pour une culture complétée par un inhibiteur et de 1,8 × 10⁸ cfu.mL⁻¹ pour une culture sans inhibiteur.

### 2.5. Quantification des bactéries produisant des bêta-lactamases

La capacité de la méthode de l'invention pour une détermination quantitative de bactéries produisant des BLSEs a été évaluée par une série de dilution d'une culture de *E.coli* BLSE⁺ de 5 × 10⁴ à 5 × 10⁷ cfu.mL⁻¹, incubées avec le céfotaxime. La courbe de calibration de la figure 5 présente une large zone de linéarité ce qui permet d'envisager la détermination quantitative des souches bactériennes productrices de BLSEs. Le plateau observé aux concentrations élevées de bactéries productrices de BLSEs suggère que la totalité de la nitrocéfine initialement présente est hydrolysée.

### 2.6. Quantification des bactéries produisant des bêta-lactamases dans des eaux usées brutes ou autoclavées

Les courbes d'étalonnage en *E*. *coli* productrices de BLSE sont établies pour les échantillons d'eaux usées brutes et les échantillons d'eaux traitées préalablement autoclavées selon la méthode décrite dans la partie 1.7.1. Ces courbes d'étalonnage sont utilisées pour quantifier les bactéries productrices de BLSE respectivement dans les eaux brutes et les eaux traitées selon la méthode de l'invention. Comme le montrent les tableaux 1 et 2, les résultats obtenus avec une méthode de l'invention concordent avec les résultats obtenus par le dénombrement de bactéries.

**Tableau 1 : Détermination des souches productrices de BLSEs (ufc.L⁻¹) dans les eaux brutes provenant de 5 stations d'épuration de Côte d'Or (A, B, C, D, E).**

| | **Dénombrement** | **Méthode décrite dans l'invention** |
|---|---|---|
| A | 2 × 10⁵ | 1,2 × 10⁵ |
| B | 7 × 10⁵ | 6 × 10⁵ |
| C | 1 × 10⁵ | 2,2 × 10⁵ |
| D | 8 × 10⁵ | 5 × 10⁵ |
| E | 3,5 × 10⁵ | 4 × 10⁵ |

**Tableau 2 : Détermination des souches productrices de BLSEs (ufc.L⁻¹) dans les eaux traitées provenant de 5 stations d'épuration de Côte d'Or (A, B, C, D, E).**

| | **Dénombrement** | **Méthode décrite dans l'invention** |
|---|---|---|
| A | 2,5 × 10³ | 1 × 10³ |
| B | 1 × 10⁴ | 2 × 10⁴ |
| C | 4 × 10³ | 4 × 10³ |
| D | 1,8 × 10³ | 5 × 10³ |
| E | 5 × 10⁴ | 4 × 10⁴ |

### 2.7. Discrimination des bêta-lactamases

Cinq souches bactériennes issues de prélèvements cliniques et produisant différents types de bêta-lactamases (pénicillinases, BLSE de type CTX-M, céphalosporinase inductible, céphalosporinase hyperproduite) ont été analysées par la méthode de l'invention.

Chaque souche bactérienne, ainsi que le témoin négatif, ont été incubés respectivement dans trois milieux de culture différents (milieux A, B et C) : le milieu A étant un milieu de culture LB, le milieu B étant un milieu de culture LB contenant 4 µg.mL⁻¹ de céfotaxime, le milieu C étant un milieu de culture LB contenant 4 µg.mL⁻¹ de céfotaxime et 100 µg.mL⁻¹ d'acide clavulanique. Après filtration des suspensions bactériennes avec des Swinnex^{®}, les membranes de filtration sur lesquelles ont été récupérées les bactéries ont été incubées dans l'obscurité pendant 10 min avec 80 µL de nitrocéfine à 500 µM.

La mesure de voltampérométrie cyclique a été mise en oeuvre à l'aide d'électrodes en carbone. Les pics de courant anodique correspondant à l'oxydation de la nitrocéfine hydrolysée pour ces trois milieux sont respectivement notés i_{A}, i_{B} et ic.

Les résultats obtenus avec les souches productrices des différentes bêta-lactamases sont rassemblés dans les figures 7A à 7F.

Les résultats de la figure 7A correspondent à la réponse d'un échantillon qui contient une souche qui ne produit pas de bêta-lactamases (négatif).

Les résultats des figures 7B et 7C correspondent à deux pénicillinases. L'activité catalytique d'une pénicillinase est caractérisée par la présence du courant anodique de la nitrocéfine hydrolysée obtenu pour les bactéries issues des milieux A et B et l'absence du courant anodique spécifique pour les bactéries issues du milieu C.

Une céphalosporinase inductible (figure 7D) est caractérisée par les pics du courant anodique de la nitrocéfine hydrolysée obtenus dans ces trois milieux correspondant aux critères suivants : i_{A}<1, i_{A}<i_{B}<i_{C}, et i_{C}>1.

Une bêta-lactamase à spectre étendu de type CTX-M (figure 7E) est caractérisée par les pics du courant anodique de la nitrocéfine hydrolysée obtenus dans ces trois milieux correspondant aux critères suivants : i_{A}>3, i_{B}>3, et i_{C}<0,2.

Une céphalosporinase hyperproduite (figure 7F) est caractérisée par les pics du courant anodique de la nitrocéfine hydrolysée obtenus dans ces trois milieux correspondant aux critères suivants : i_{A}>3, i_{A}>i_{B}>i_{C}, et i_{C}>1.

Un antibiogramme par diffusion en gélose et la méthode ampérométrique de l'invention sont respectivement mis en oeuvre pour discriminer les bactéries produisant les bêta-lactamases dans des échantillons d'hémocultures. Comme le montre les résultats rassemblés dans le tableau 3 ci-après, le taux de recouvrement de la méthode de la présente invention est de 100% avec l'antibiogramme.

**Tableau 3 : Comparaison des résultats obtenus après la réalisation d'un antibiogramme (ATB) et la mise en oeuvre de la méthode électrochimique de l'invention pour la discrimination des souches productrices de bêta-lactamases dans des bouillons d'hémocultures.**

| **n° Echantillon** | **Flore** | **ATB** | **Electrochimie*** |
|---|---|---|---|
| 035 | *Escherichia coli* | C3G et autres : S | i_{A} = 0,15 - i_{B} = 0,13 - i_{C} = 0,15 |
| | | | Pas de bêta-lactamase |
| | | | C3G et autres : S |
| 034 | *Escherichia coli* | C3G : R (BLSE) | i_{A} = 5,1 - i_{B} = 3,1 - i_{C} = 0,17 |
| | | | BLSE |
| | | | C3G : R |
| 033 | *Escherichia coli* | C3G : R (BLSE) | i_{A} = 3,7 - i_{B} = 3,1 - i_{C} = 0,18 |
| | | | BLSE |
| | | | C3G : R |
| 030 | *Proteus mirabilis* | C3G: S (Pénicillinase ) | i_{A} = 2 - i_{B} = 0,3 - i_{C} = 0,18 |
| | | | Pénicillinase |
| | | | C3G: S |
| 029 | *Escherichia coli* | C3G: S (Pénicillinase) | i_{A} = 1,3 - i_{B} = 2,2- i_{C} = 0,16 |
| | | | Pénicillinase |
| | | | C3G : S |
| 026 | *Escherichia coli* | C3G et autres : S (pas de bêta-lactamase) | i_{A} = 0,16 - i_{B} = 0,14 - i_{C} = 0,14 |
| | | | Pas de bêta-lactamase |
| | | | C3G et autres : S |
| 025 | *Escherichia coli* | C3G: S (Pénicillinase) | i_{A} = 1,4 - i_{B} = 1,5 - i_{C} = 0,2 |
| | | | Pénicillinase |
| | | | C3G: S |
| 024 | *Escherichia coli* | C3G: S (Pénicillinase) | i_{A} = 0,7 - i_{B} = 1,5 - i_{C} = 0,15 |
| | | | Pénicillinase |
| | | | C3G : S |
| 019 | *Escherichia coli* | C3G et autres : S | i_{A} = 0,17 - i_{B} = 0,17 - i_{C} = 0,16 |
| | | | Pas de bêta-lactamase |
| | | | C3G et autres : S |
| 005 | *Escherichia coli* | C3G: S (Pénicillinase) | i_{A} = 0,42 - i_{B} = 0,96 - i_{C} = 0,18 |
| | | | Pénicillinase |
| | | | C3G : S |

| | | | |
|---|---|---|---|
| * i_{A}, i_{B}, et i_{C} (µA) | | | |

Un antibiogramme par diffusion sur gélose et la méthode ampérométrique de l'invention sont également mis en oeuvre pour discriminer des bactéries productrices de bêta-lactamases, issues de prélèvements biologiques variés, après leur isolement sur une gélose lactosée de Drigalski. Les résultats rassemblés dans le Tableau 4 indiquent que le taux de recouvrement de la méthode de la présente invention est de 100% avec l'antibiogramme.

**Tableau 4 : Comparaison des résultats obtenus après la réalisation d'un antibiogramme (ATB) et la mise en oeuvre de la méthode électrochimique de l'invention pour la discrimination des souches productrices de bêta-lactamases préalablement isolées sur milieu Drigalski.**

| **n° Echantillon** | **Flore** | **ATB** | **Electrochimie*** |
|---|---|---|---|
| 018 | *Enterobacter cloacae* | C3G: S Céphalosporinase inductible | i_{A} = 0,23 - i_{B} = 0,27- i_{C} = 1,3 |
| | | | Céphalosporinase inductible C3G : |
| | | | S |
| 014 | *Escherichia coli* | C3G : R (BLSE) | i_{A} = 5,5 - i_{B} = 5,6 - i_{C} : 0,23 |
| | | | BLSE |
| | | | C3G : R |
| 013 | *Escherichia coli* | C3G : R (BLSE) | i_{A} = 5,8 - i_{B} = 5,6 - i_{C} = 0,13 |
| | | | BLSE |
| | | | C3G : R |
| 012 | *Escherichia coli* | C3G : R (BLSE) | i_{A} = 5,0- i_{B} = 3,1 - i_{C} = 0,19 |
| | | | BLSE |
| | | | C3G : R |
| 008 | *Klebsiella oxytoca* (urine) | C3G: S (Pénicillinase) | i_{A} = 0,25 - i_{B} = 0,55 - i_{C} = 0,24 |
| | | | Pénicillinase |
| | | | C3G: S |
| 007 | *Proteus mirabilis* (os) | C3G: S (Pénicillinase) | i_{A} = 0,14 - i_{B} = 1,3- i_{C} = 0,16 |
| | | | Pénicillinase |
| | | | C3G: S |
| 004 | *Proteus mirabilis* (pus) | C3G: S (Pénicillinase) | i_{A} = 2,8 - i_{B} = 3,4 - i_{C} = 0,4 |
| | | | Pénicillinase |
| | | | C3G: S |

| | | | |
|---|---|---|---|
| *i_{A}, i_{B}, et i_{C} (µA) | | | |

### Exemple II : Identification des bactéries résistantes aux C3G utilisant le composé HMRZ-86 comme substrat

### 1. Matériel et méthodes :

### 1.1. Réactifs et solutions

Le composé HMRZ-86 a été synthétisé et fourni par Kanto Chemical (Japon). Une solution mère à 10⁻² M est préparée dans du DMSO et conservée sous forme d'aliquotes de 50 µL à -20°C.

Les souches utilisées dans cet exemple et rassemblées dans le tableau 5 ci-dessous proviennent de la collection de souches cryoconservées de l'INRA de Dijon.

**Tableau 5**

| **Espèces** | **Résistance aux bêta-lactamines** | |
|---|---|---|
| | Phénotype | Génotype |
| *Escherichia coli* | Type sauvage | Aucun gène |
| *Escherichia coli* | Pénicillinase | |
| Citrobacter freundii | Céphalosporinase Inductible | |
| *Escherichia coli* | BLSE | CTX-M 15 |
| *Klebsiella pneumoniae* | BLSE | CTX-M 15 |
| *Serratia marcescens* | Céphalosporinase Hyperproduite | |
| *Klebsiella pneumoniae* | Carbapénémase | OXA 48 -CTXM |

### 1.2. Identification des souches résistantes aux C3G dans des cultures liquides

Chaque souche cryoconservée est mise en culture dans 10 mL de milieu LB à 37°C jusqu'à obtention de la phase stationnaire.

Un volume de 50 µL de culture liquide est incubé en présence de 50 µL d'une solution de HMRZ-86 à 0,5 mM dans PBS à 37°C sous agitation pendant 30 min. 40 µL du mélange réactionnel sont prélevés et transférés sur la surface d'un capteur de carbone sérigraphié (Dropsens, DRP-110) préalablement connecté à un potentiostat portable STAT 8000P (Dropsens) alimenté par la connexion USB de l'ordinateur et piloté par le logiciel DropView 8400. Les mesures de voltammétrie linéaire sont réalisées en effectuant un balayage linéaire de potentiel à partir de -0,1 V jusqu'à 1,2 V à une vitesse de 50 mV.s⁻¹. Le courant de pic apparaissant dans l'intervalle de potentiel +0,2 -+0,55 V *vs.* Ag/AgCl, lié à l'hydrolyse du cycle bêta-lactame du HMRZ-86, est choisi comme réponse analytique. Si l'intensité du pic (*i*) possède une valeur supérieure à 500 nA, l'échantillon contient une souche capable de dégrader les C3G.

### 1.3. Réalisation d'une courbe d'étalonnage de souches d'E.coli productrices de BLSE dans des échantillons d'eau usées autoclavées

Le protocole décrit en 1.7.1. de l'Exemple I a été mise en oeuvre avec une seule condition de culture (LB supplémenté avec du céfotaxime à 4 µg.mL⁻¹). Les filtres sont incubés avec 90 µL de HMRZ-86 (0,25 mM dans PBS) à 37°C pendant 30 min. Les mesures sont réalisées par voltammétrie linéaire comme mentionnés précédemment (paragraphe 1.2). Le courant de pic apparaissant dans l'intervalle de potentiel +0,2 - +0,55 V *vs.* Ag/AgCl, lié à l'hydrolyse du cycle bêta-lactame du HMRZ-86, est choisi comme réponse analytique. Son intensité (*i*) est proportionnelle à la quantité de souches résistantes aux C3G présente dans l'échantillon.

### 2. Résultats

### 2.1. Caractérisation électrochimique du HMRZ-86 et de ses formes hydrolysées

Une solution de HMRZ-86 a été incubée avec différentes souches bactériennes résistantes ou sensibles aux C3G, c'est-à-dire respectivement capables ou non d'hydrolyser la molécule de HMRZ-86.

Les réponses voltammétriques du HMRZ-86 et de ses formes hydrolysées sont présentées (Figure 8). Les voltammogrammes enregistrés pour le HMRZ-86 incubé avec les souches sensibles aux C3G (non productrice de bêta-lactamase, productrices de pénicillinase ou de céphalosporinase inductible) possèdent tous le même profil avec deux vagues d'oxydation de très faible intensité vers +0,2 et +0,4 V *vs.* Ag/AgCl qui disparaissent au profit de pics d'oxydation bien définis pour les formes hydrolysées du HMRZ-86. Plus précisément, l'hydrolyse du HMRZ-86 par les souches productrices de BLSE et de carbapénémases conduit à un produit qui présente un pic d'oxydation P1 (Figure 8) bien spécifique vers + 0,25 V *vs.* Ag/AgCl, tandis que le produit résultant de l'hydrolyse du HMRZ-86 par une souche productrice de céphalosporinase hyperproduite s'oxyde spécifiquement vers ∼ + 0,5 V *vs.* Ag/AgCl pour donner un pic P2 (Figure 8). Ainsi, la présence des pics P1 ou P2 indique celle du HMRZ-86 hydrolysé et peut-être choisie comme réponse analytique pour mettre en évidence les souches résistantes aux C3G. De plus, le pic P2 permet d'identifier spécifiquement les souches productrices de céphalosporinases hyperproduites.

### 2.2. Quantification des souches résistantes aux C3G : exemple de courbes de calibration de souches productrices de BLSE dans de l'eau usée autoclavée

La capacité de la méthode de l'invention pour une détermination quantitative des bactéries résistantes aux C3G avec le substrat HMRZ-86 a été évaluée en appliquant la courbe d'étalonnage établie selon le protocole 1.3. décrit dans le présent exemple. La figure 9 rassemble les courbes d'étalonnage obtenue pour deux souches d*'E.coli* productrices de BLSE (type CTX-M15) inoculées dans des échantillons d'eaux usées préalablement autoclavées. Avec une zone de linéarité comprise entre 10 et 1000 bactéries filtrées, cette méthode permet d'envisager la quantification de souches résistantes aux C3G avec une bonne sensibilité.

### Exemple III : Identification des bactéries résistantes aux carbapénèmes

### 1. Matériel et méthodes :

### 1.1. Réactifs

Le substrat désigné ci-après « Carba-S » et son diluant correspondent respectivement aux réactifs R2 et R3 du kit β Carba^{™} Test commercialisé par Biorad. La solution Carba-S est préparée en ajoutant 550 µL de R3 dans R2.

Les souches utilisées dans cet exemple et rassemblées dans le tableau 6 ci-dessous proviennent de la collection de souches cryoconservées de l'INRA de Dijon.

**Tableau 6**

| **Espèces** | **Résistance aux bêta-lactamines** | |
|---|---|---|
| | Phénotype | Génotype |
| *Klebsiella pneumoniae* | BLSE | CTX-M 15 |
| *Serratia marcescens* | Céphalosporinase Hyperproduite | |
| *Klebsiella pneumoniae* | Carbapénémase | OXA 48 -CTXM |
| *Escherichia coli* | Carbapénémase | OXA-48 |
| *Klebsiella pneumoniae* | Carbapénémase | KPC-2 |
| *Klebsiella pneumoniae* | Carbapénémase | NDM-1 |

### 1.2. Identification des souches résistantes aux carbapénèmes à partir de colonies isolées

Chaque souche cryoconservée est mise en culture sur une gélose Muller Hinton à 37°C.

Une oëse de 1 µL est incubée en présence de 30 µL de la solution Carba-S à 37°C sous agitation pendant 30 min. 40 µL du mélange réactionnel sont prélevés et transférés sur la surface d'un capteur de carbone sérigraphié (Dropsens, DRP-110) préalablement connecté à un potentiostat portable STAT 8000P (Dropsens) alimenté par la connexion USB de l'ordinateur et piloté par le logiciel DropView 8400. Les mesures de voltammétrie linéaire sont réalisées en effectuant un balayage linéaire de potentiel à partir de -0,1 V jusqu'à 1,2 V à une vitesse de 50 mV.s⁻¹. Le courant de pic (*i*) apparaissant vers + 0,3-0,8 V *vs.* Ag/AgCl, lié à l'hydrolyse du Carba-S, est choisi comme réponse analytique. La présence du pic (*i*) avec une intensité supérieure à 200 nA permet de conclure sur la présence d'une souche productrice de carbapénémase.

### 2.2. Résultats :

### 2.1. Caractérisation électrochimique du substrat Carba-S et de ses formes hydrolysées à partir de colonies isolées

La solution de Carba-S a été incubée avec différentes souches bactériennes sensibles ou résistantes aux carbapénèmes, c'est-à-dire respectivement capables ou non de dégrader la molécule de Carba-S et analysée par voltammétrie linéaire avec des capteurs sérigraphiés.

Les réponses voltammétriques enregistrées pour des colonies sensibles aux carbapénèmes (productrices de BLSE et de céphalosporinases hyperproduites) et des colonies productrices de carbapénémases de type OXA 48, OXA 48 + CTXM, KPC-2et NDM-1 sont respectivement présentées (Figure 10A, 10B, 10C et 10D). La comparaison des voltammogrammes montre que seules les courbes enregistrées pour les souches productrices de carbapénémases présentent une à trois vagues d'oxydation avec des pics bien définis dans un intervalle de potentiels de +0,2 à +0,8 V vs. Ag/AgCl. Ainsi, grâce à une mesure voltammétrique, il est possible de distinguer le Carba-S de ses produits de dégradation et donc de mettre en évidence la présence de souches productrices de carbapénémases.

### Exemple IV: Résultats comparatifs

### Test comparatif 1

La méthode électrochimique de l'invention a été appliquée à l'analyse d'un panel de 40 souches productrices de différentes β-lactamases et comparée avec le β-Lacta^{™} test (Biorad) dont le principe repose sur une détection colorimétrique qualitative des souches résistantes aux C3G. Les résultats rassemblés dans le Tableau 7 ont été obtenus après 30 min d'incubation des bactéries avec le substrat HMRZ-86 pour les deux méthodes. En s'appuyant sur l'interprétation des résultats proposée par le fournisseur, le β-Lacta^{™} Test permet l'identification des souches résistantes aux C3G avec un taux de concordance de 68 % tandis qu'avec la méthode électrochimique de l'invention un taux de concordance de 95 % a été obtenu. Contrairement à la colorimétrie, l'ampérométrique offre l'avantage de mesurer une valeur de courant chiffrée ce qui, d'une part, évite toute interprétation subjective du résultat (couleur intermédiaire), et d'autre part, permet la traçabilité du résultat.

Tableau 7 : Comparaison des résultats obtenus avec la méthode électrochimique de l'invention pour des cultures liquides avec ceux obtenus avec le β-Lacta^{™} Test mis en oeuvre selon les recommandations du fournisseur avec des colonies isolées. Dans les deux cas, les lectures ont été réalisées après 30 min d'incubation avec le substrat HMRZ-86.

**Tableau 7**

| **Résistance aux bêta-lactamines** | | **Espèce** | **β-Lacta^{™} Test^{a}** | **Quantité de bactéries analysées (ufc)** | **Test électrochi mique^{b}** |
|---|---|---|---|---|---|
| Phénotype | Génotype | | Couleur | | Intensité (nA) |
| Bêta-lactamase négative | | *E. coli* K12 | Jaune | 7,3 × 10⁷ | 302 |
| Bêta-lactamase négative | | *P. mirabilis* 11747 | Jaune | - | 322 |
| Pénicillinase bas niveau | | *P. mirabilis* 11768 | Jaune | - | 233 |
| Pénicillinase bas niveau | | *K. pneumoniae* 11770 | Jaune | 7,15 × 10⁷ | 365 |
| Pénicillinase bas niveau | | *K. oxytoca* 11779 | Jaune | 5,35 × 10⁷ | 412 |
| Pénicillinase bas niveau | | *E. coli* 11781 | Jaune | 3,8 × 10⁷ | 377 |
| Pénicillinase hyperproduite | | *E. coli* 11746 | Jaune | 3,75 × 10⁷ | 380 |
| Pénicillinase hyperproduite | | *E. coli* 11765 | Jaune | 3,1 × 10⁷ | 323 |
| Pénicillinase hyperproduite | | *E. coli* 11771 | Jaune | 7,05 × 10⁷ | 362 |
| Pénicillinase hyperproduite | | *E. coli* 11780 | Jaune | 5,15 × 10⁷ | 425 |
| Céphalosporinase inductible | | C. *freundii* 11745 | Jaune | 2,95 × 10⁷ | 369 |
| Céphalosporinase inductible | | *E. coli* 11748 | Jaune | 4,65 × 10⁷ | 424 |
| Céphalosporinase inductible | | *E. coli* 11766 | Jaune | 7,65 × 107 | 408 |
| Céphalosporinase inductible | | *E. aerogenes* 11772 | Jaune | 9,5 × 10⁷ | 362 |
| Céphalosporinase inductible | | *E. coli* 11776 | Jaune | 3,7 × 10⁷ | 446 |
| Céphalosporinase hyperproduite | | *P. aeruginosa* 11774 | Orange | 5,3 × 10⁷ | **2160** |
| Céphalosporinase hyperproduite | | *E. aerogenes 11775* | Jaune | 9,45 × 10⁷ | **501** |
| Céphalosporinase hyperproduite | | *P. aeruginosa 11778* | Jaune | 7,4 × 10⁷ | **2278** |
| Céphalosporinase hyperproduite | | P. *aeruginosa 11782* | Jaune | 5,95 × 10⁷ | **1778** |
| Céphalosporinase hyperproduite | | *E. cloacae* 11784 | Orange | 7,95 × 10⁷ | **569** |
| Céphalosporinase hyperproduite | | S. *marcescens* 11787 | Orange | 8,55 × 10⁷ | **3922** |
| Céphalosporinase hyperproduite | | *E. cloacae* 11790 | Orange | 5,15 × 10⁷ | **686** |
| Céphalosporinase hyperproduite | | *E. coli* 11791 | Jaune | 5,15 × 10⁷ | **816** |
| Céphalosporinase hyperproduite | | *E. cloacae* 11794 | Orange | 7,95 × 10⁷ | **842** |
| BLSE | TEM 24 | *E. aerogenes* 11796 | Orange | 5,7 × 10⁷ | **1249** |
| BLSE | CTX-M-1 | *E. coli* 11810 | Rouge | 8,05 × 10⁷ | **2544** |
| BLSE | CTX-M-15 | *K. pneumoniae* 11812 | Rouge | 5,35 × 10⁷ | **3494** |
| BLSE | CTX-M-2 | *E. coli* 11817 | Rouge | 5,25 × 10⁷ | **1472** |
| BLSE | CTX-M-1 | *E. coli* 11818 | Rouge | 4,25 × 10⁷ | **3583** |
| BLSE | SHV-12 | *E. coli* 11820 | Rouge | 5,95 × 10⁷ | **1249** |
| BLSE | CTX-M-27 | *E. coli* 11828 | Rouge | 4,15 × 10⁷ | **2640** |
| BLSE | CTX-M-9 | *E. coli* 11833 | Rouge | 4,7 × 10⁷ | **2455** |
| BLSE | TEM-12 | *E. coli* 11834 | Jaune | 2,85 × 10⁷ | 418 |
| BLSE | CTX-M-14 | *E. coli* 11863 | Rouge | 2,95 × 10⁷ | **2544** |
| Carbapénémase | OXA-48 - CTX-M | *K. pneumoniae* 11754 | Rouge | 6,4 × 10⁷ | **3520** |
| Carbapénémase | OXA-48 - CTX-M | *E. coli* 11755 | Rouge | 6,75 × 10⁷ | **3509** |
| Carbapénémase | OXA-48 | *E. coli* 11757 | Orange | 9,45 × 10⁷ | **625** |
| Carbapénémase | OXA-48 | *K. pneumoniae* 11762 | Orange | 4,95 × 10⁷ | 476 |
| Carbapénémase | KPC-2 | *K. pneumoniae* 11839 | Rouge | 3,2 × 10⁷ | **1567** |
| Carbapénémase | NDM-1 | *K. pneumoniae* 11842 | Rouge | 3,55 × 10⁷ | **1257** |

| | | | | | |
|---|---|---|---|---|---|
| ^{a}. Couleur jaune ou orange : résultat négatif (absence de souches résistantes aux C3G). Couleur rouge : résultat positif (présence de souches résistantes aux C3G) ^{b}. i < 490 nA : résultat négatif (absence de souches résistantes aux C3G). i > 490 nA : résultat positif (présence de souches résistantes aux C3G). La valeur de i = 490 nA correspond à la moyenne des 12 réponses obtenues pour les souches productrices de pénicillinases et de céphalosporinases inductibles à laquelle a été ajoutée 3 fois l'écart-type. | | | | | |

### Test comparatif 2

La méthode électrochimique de l'invention a été appliquée à l'analyse d'un panel de 28 souches productrices de BLSE, céphalosporinase hyperproduite et de carbapénémases et comparée avec le β-Carba^{™} test (Biorad) dont le principe repose sur une détection colorimétrique qualitative des souches résistantes aux carbapénèmes. Les résultats rassemblés dans le Tableau 8 ont été obtenus après 30 min d'incubation d'une oëse d'1 µL de colonies isolées avec le substrat Carba-S pour les deux méthodes. En s'appuyant sur l'interprétation des résultats proposée par le fournisseur, le β-Carba^{™} Test permet l'identification des souches résistantes aux carbapénèmes avec un taux de concordance de 43 % tandis qu'avec la méthode électrochimique de l'invention un taux de concordance de 100 % a été obtenu. Là encore, grâce à une mesure de courant chiffrée, la méthode électrochimique de l'invention évite toute interprétation subjective du résultat (couleur intermédiaire), et permet l'obtention d'un résultat fiable beaucoup rapidement que la colorimétrie.

Tableau 8 : Comparaison des résultats obtenus pour l'analyse de colonies isolées avec la méthode électrochimique de l'invention et avec le β-Carba^{™} Test mis en oeuvre selon les recommandations du fournisseur. Dans les deux cas, les lectures ont été réalisées après 30 min d'incubation avec le substrat Carba-S.

**Tableau 8**

| **Résistance aux bêta-lactamines** | | **Espèce** | **β- Carba^{™} Test^{a}** | **Test électrochimiq ue^{b}** |
|---|---|---|---|---|
| Phénotype | Génotype | | Couleur | Intensité (nA) |
| BLSE | CTX-M-15 | *E. coli* 11808 | Jaune | 77 |
| BLSE | CTX-M-14 | *E. coli* 11806 | Jaune | 148 |
| BLSE | CTX-M-1 | *E. coli* 11810 | Jaune | 132 |
| BLSE | CTX-M-15 | *K. pneumoniae* 11812 | Jaune | 128 |
| BLSE | CTX-M-2 | *E. coli* 11817 | Jaune | 147 |
| BLSE | CTX-M-1 | *E. coli* 11818 | Jaune | 118 |
| BLSE | CTX-M-14 | *E. coli* 11823 | Jaune | 137 |
| BLSE | CTX-M-14 | *E. coli* 11824 | Jaune | 128 |
| BLSE | CTX-M-14 | *E. coli* 11826 | Jaune | 130 |
| Céphalosporinase hyperproduite | | *E. coli* 11791 | Jaune | 93 |
| Carbapénémase | OXA-48 - CTX-M | *K. pneumoniae* 11749 | Orange | **4103** |
| Carbapénémase | OXA-48 - CTX-M | *K. pneumoniae* 11752 | Jaune | **2281** |
| Carbapénémase | OXA-48 - CTX-M | *E. coli* 11753 | Jaune | **1055** |
| Carbapénémase | OXA-48 - CTX-M | *K. pneumoniae* 11754 | Jaune | **1129** |
| Carbapénémase | OXA-48 - CTX-M | *E. coli* 11755 | Orange | **1836** |
| Carbapénémase | OXA-48 - CTX-M | *K. pneumoniae* 11756 | Orange | **2616** |
| Carbapénémase | OXA-48 - CTX-M | *E. coli* 11764 | Jaune | **1281** |
| Carbapénémase | OXA-48 | *E. coli* 11750 | Jaune | **741** |
| Carbapénémase | OXA-48 | *E. coli* 11757 | Jaune | **716** |
| Carbapénémase | OXA-48 | *E. coli* 11758 | Orange | **1458** |
| Carbapénémase | OXA-48 | *K. pneumoniae* 11761 | Jaune | **1191** |
| Carbapénémase | OXA-48 | *K. pneumoniae* 11762 | Jaune | **791** |
| Carbapénémase | OXA-48 | *E. coli* 11763 | Jaune | **1033** |
| Carbapénémase | KPC-2 | *K. pneumoniae* 11839 | Rouge | **3437** |
| Carbapénémase | KPC-2 | *K. pneumoniae* 11840 | Orange | **3427** |
| Carbapénémase | KPC-2 | *K. pneumoniae* 11841 | Rouge | **3731** |
| Carbapénémase | NDM-1 | *K. pneumoniae* 11842 | Orange | **1761** |

| | | | | |
|---|---|---|---|---|
| ^{a}. Couleur jaune ou orange : résultat négatif (absence de production de carbapénémases). Couleur orange franc ou rouge ou pourpre : résultat positif (présence de production de carbapénémases) ^{b}. i < 195 nA : résultat négatif (absence de production de carbapénémases). i > 195 nA : résultat positif (présence ). La valeur de i=195 nA correspond à la moyenne des 10 réponses obtenues pour les souches non productrices de carbapénémases à laquelle a été ajoutée 3 fois l'écart-type. | | | | |

## Revendications

1. Méthode pour déterminer *in vitro* la présence de bactéries produisant des bêta-lactamases dans un échantillon susceptible de contenir lesdites bactéries, ladite méthode comprenant les étapes suivantes:
(a) incuber ledit échantillon dans un milieu contenant un substrat des bêta-lactamases, possédant des propriétés électrochimiques, notamment une bêta lactamine possédant des propriétés électrochimiques, et
(b) appliquer un moyen d'analyse électrochimique pour déterminer la présence du substrat des bêta-lactamases après avoir été hydrolysés.

2. Méthode selon la revendication 1, **caractérisée en ce que** ledit moyen d'analyse électrochimique est une mesure potentiométrique, une mesure d'impédancemétrie, une mesure coulométrique ou une mesure ampérométrique.

3. Méthode selon la revendication 1 ou 2, **caractérisée en ce que** ledit substrat des bêta-lactamases est une bêta-lactamine, notamment choisi dans le groupe comprenant la nitrocéfine, le composé HMRZ-86 (Isomère E de l'acide (6R,7R)-trifluoroacétate 7-[[2-(2-amino-4-thiazolyl)-2-[(1-carboxy-1-méthyléthoxy)imino]acétyl]amino]-1-aza-3-[2-(2,4-dinitrophényl)éthényl]-8-oxo-5-thiabicyclo[4.2.0]oct-2-ène-2-carboxylique), ou le substrat (réactif R2) du kit β CARBA^{™}.

4. Méthode selon l'une quelconque des revendications 1 à 3, **caractérisée en ce que** ladite méthode comprend les étapes suivantes :
(a) incuber un échantillon susceptible de contenir lesdites bactéries dans un milieu contenant un substrat des bêta-lactamases, notamment une bêta-lactamine, possédant des propriétés électrochimiques,
(b) appliquer une mesure ampérométrique respectivement au susdit milieu obtenu à la fin de l'étape (a) et à un contrôle négatif,
(c) déterminer la présence des bactéries produisant des bêta-lactamases par la comparaison de la valeur de l'intensité du courant anodique correspondant à l'oxydation du substrat hydrolysé, notamment de la bêta-lactamine hydrolysée, mesurée pour le susdit milieu obtenu à la fin de l'étape (a), avec la valeur de l'intensité du courant anodique mesurée pour le contrôle négatif.

5. Méthode selon la revendication 4 pour déterminer et quantifier les bactéries produisant des bêta-lactamases dans un échantillon susceptible de contenir lesdites bactéries, ladite méthode comprenant en outre, après l'étape (c), une étape (d) consistant à comparer la valeur de l'intensité du courant anodique mesurée pour ledit milieu obtenu à la fin de l'étape (a) avec une courbe d'étalonnage établie dans les mêmes conditions.

6. Méthode selon l'une quelconque des revendications 1 à 3 permettant en outre de discriminer le type de bêta-lactamases choisies parmi les pénicillinases, les bêta-lactamases à spectre étendu, les céphalosporinases inductibles, les céphalosporinases hyperproduites et les carbapénèmases, produites par les susdites bactéries dans un échantillon susceptible de les contenir, ladite méthode comprenant les étapes suivantes :
(a) incuber parallèlement une fraction de susdit échantillon dans :
un milieu de culture A, un milieu de culture B et un milieu de culture C :
- le milieu de culture A étant un milieu de culture de base,
- le milieu de culture B étant un milieu de culture de base complété par une céphalosporine de 3^{ème} génération, et
- le milieu de culture C étant un milieu de culture de base complété par une céphalosporine et un inhibiteur de pénicillinases, et
éventuellement dans un milieu de culture B', un milieu de culture C', un milieu de culture D, un milieu de culture E :
- le milieu de culture B' étant un milieu de culture de base complété par une céphalosporine de 3^{ème} génération différente de celle présente dans le milieu B ;
- le milieu de culture C' étant le milieu de culture B' complété par un inhibiteur de pénicillinases ;
- le milieu de culture D étant un milieu de culture de base complété par une céphalosporine et un inhibiteur des céphalosporinases, et
- le milieu de culture E étant un milieu de culture de base complété par un carbapénème,
(b) incuber parallèlement les susdits milieux de culture obtenus à la fin de l'incubation de l'étape (a) dans un milieu contenant la nitrocéfine;

7. Méthode selon la revendication 6 pour déterminer la présence des bactéries produisant une bêta-lactamase à spectre étendu, ladite méthode comprenant les étapes suivantes:
(a) incuber parallèlement une fraction de susdit échantillon dans un milieu de culture A, un milieu de culture B et un milieu de culture C, et éventuellement dans un milieu de culture B' et un milieu de culture C', tels que définis selon la revendication 6,
(b) incuber parallèlement les susdits milieux de culture obtenus à la fin de de l'incubation de l'étape (a) dans un milieu contenant la nitrocéfine,

8. Méthode selon la revendication 6, pour déterminer et discriminer les bactéries produisant des bêta-lactamases dans un échantillon susceptible de les contenir, comprenant les étapes suivantes :
(a) incuber parallèlement une fraction du susdit échantillon dans un milieu de culture A, un milieu de culture B, un milieu de culture C, un milieu de culture D, un milieu de culture E, éventuellement un milieu B' et un milieu C', tels que définis selon la revendication 6,
(b) incuber parallèlement les susdits milieux de culture obtenus à la fin de l'incubation de l'étape (a) dans un milieu contenant la nitrocéfine,
(c) appliquer une mesure ampérométrique respectivement aux susdits milieux obtenus à la fin de l'étape (b) et à un contrôle négatif,
(d) déterminer la présence de bactéries produisant des bêta-lactamases dans ledit échantillon par la comparaison de la valeur de l'intensité du courant anodique correspondant à l'oxydation de nitrocéfine hydrolysée obtenu pour la fraction cultivée dans le milieu de culture A avec la valeur de l'intensité du courant obtenu pour le contrôle négatif, et
(e) discriminer le type de bêta-lactamases produites par les susdites bactéries dans ledit échantillon, par la comparaison des valeurs respectives de l'intensité du susdit courant anodique obtenues pour les fractions cultivées parallèlement dans les milieux de culture A, B, C, D et E, éventuellement B' et C' avec les valeurs respectives obtenues pour une souche bactérienne de référence.

9. Méthode selon la revendication 8, comprenant en outre une étape (f) après l'étape (e) permettant de quantifier des bactéries produisant la bêta-lactamase discriminée dans l'étape (e) par comparaison de la valeur de l'intensité du courant anodique correspondant à la nitrocéfine hydrolysée par ladite bêta-lactamase avec une courbe d'étalonnage établie dans les mêmes conditions.

10. Méthode selon l'une quelconque des revendications 6, 8-9, pour discriminer le type des bêta-lactamases et éventuellement préciser la sous-famille de carbapénèmases produites par les susdites bactéries dans un échantillon susceptible de les contenir, ladite méthode comprenant les étapes suivantes :
(a) incuber parallèlement une fraction du susdit échantillon dans un milieu de culture A, un milieu de culture B, un milieu de culture C, un milieu de culture D, un milieu de culture E, et un milieu de culture F, et éventuellement un milieu de culture B' et un milieu de culture C',
les milieux A, B, B', C, C', D, E étant tels que définis selon la revendication 6,
le milieu de culture F étant un milieu de culture de base complété par un carbapénème et un inhibiteur spécifique à une sous-famille de carbapénèmases;
(b) incuber parallèlement les susdits milieux de culture obtenus à la fin de l'incubation de l'étape (a) dans un milieu contenant la nitrocéfine,
(c) appliquer une mesure ampérométrique respectivement aux susdits milieux obtenus à la fin de l'étape (b) et à un contrôle négatif,
(d) déterminer la présence de bactéries produisant des bêta-lactamases dans ledit échantillon par la comparaison de la valeur de l'intensité du courant anodique correspondant à l'oxydation de la nitrocéfine hydrolysée obtenue pour la fraction cultivée dans le milieu de culture A avec la valeur de l'intensité du courant obtenue pour le contrôle négatif,
(e) discriminer le type de bêta-lactamases produites par les susdites bactéries dans ledit échantillon, par la comparaison des valeurs respectives de l'intensité du susdit courant anodique obtenues pour les fractions cultivées parallèlement dans les milieux de culture A, B, C, D, E et F, et éventuellement les milieux de culture B' et C', avec les valeurs respectives obtenues pour une souche bactérienne de référence.

11. Méthode selon l'une quelconque des revendications 1 à 10, **caractérisée en ce que** l'intensité du courant anodique correspondant à l'oxydation de la nitrocéfine hydrolysée dans un tampon de pH neutre est mesurée dans un intervalle de potentiels compris entre + 0,1 V et + 0,5 V *vs.* Ag/AgCl, notamment entre + 0,2 V et + 0,4 V *vs.* Ag/AgCl, plus particulièrement entre + 0,23V et + 0,33V vs. Ag/AgCl.

12. Méthode selon l'une quelconque des revendications 6 à 10, **caractérisée en ce qu'**une céphalosporine de 3^{ème} génération est choisie dans le groupe comprenant le céfotaxime, la ceftazidime et la ceftriaxone ; qu'un inhibiteur de pénicillinase est l'acide clavulanique, le tazobactam ou le sulbactam ; que ledit inhibiteur de céphalosporinases est la cloxacilline ; qu'un carbapénème est l'ertapémème, l'imipénème ou le méropénème ; qu'un inhibiteur de carbapénèmases est choisi parmi l'EDTA, l'acide mercaptoacétique, l'acide boronique, ou l'acide clavulanique.

13. Méthode selon l'une quelconque des revendications 1 à 5, **caractérisée en ce que** ladite méthode est utilisée pour déterminer et quantifier *in vitro* la présence de bactéries résistantes à une céphalosporine de 3^{ème} génération et que le milieu utilisé dans l'étape (a) contient une bêta-lactamine hydrolysée uniquement par les bêta-lactamases à spectre étendu, les céphalosporinases hyperproduites et les carbapénèmases.

14. Méthode selon l'une quelconque des revendications 1 à 5, **caractérisée en ce que** ladite méthode est utilisée pour déterminer et quantifier *in vitro* la présence de bactéries productrices de carbapénémases et que le milieu utilisé dans l'étape (a) contient un substrat hydrolysé uniquement par les carbapénèmases.

15. Méthode selon l'une quelconques des revendications 1 à 14, **caractérisée en ce que** ledit échantillon est choisi parmi un prélèvement biologique, un échantillon d'origine environnementale, ou un échantillon alimentaire.

## Patentansprüche

1. Verfahren zur in-vitro-Bestimmung des Vorhandenseins von Beta-Lactamasen produzierenden Bakterien in einer Probe, die möglicherweise solche Bakterien enthält, wobei das Verfahren die folgenden Schritte umfasst:
(a) Inkubation der Probe in einem Medium, das ein Beta-Lactamasen-Substrat mit elektrochemischen Eigenschaften enthält, insbesondere ein Beta-Lactam mit elektrochemischen Eigenschaften, und
(b) Anwendung eines elektrochemischen Analysemittels zur Bestimmung des Vorhandenseins des Beta-Lactamasen-Substrats nach dessen Hydrolyse.

2. Verfahren nach Anspruch 1, **dadurch gekennzeichnet, dass** das elektrochemische Analysemittel eine potentiometrische Messung, eine impedanzmetrische Messung, eine coulometrische Messung oder eine amperometrische Messung ist.

3. Verfahren nach Anspruch 1 oder 2, **dadurch gekennzeichnet, dass** das Beta-Lactamasen-Substrat ein Beta-Lactam ist, insbesondere ausgewählt aus der Gruppe umfassend Nitrocefin, die Verbindung HMRZ-86 (E-Isomer der Säure (6R,7R)-Trifluoracetat-7-[[2-(2-Amino-4-thiazolyl)-2-[(1-carboxy-1-methylethoxy)imino]acetyl]amino]-1-aza-3-[2-(2,4-dinitrophenyl)ethenyl]-8-oxo-5-thiabicyclo[4.2.0]oct-2-en-2-carboxyl), oder das Substrat (Reagenz R2) des *β* CARBA^{™}-Kits.

4. Verfahren nach einem der Ansprüche 1 bis 3, **dadurch gekennzeichnet, dass** das Verfahren die folgenden Schritte umfasst:
(a) Inkubation einer Probe, die möglicherweise die genannten Bakterien enthält, in einem Medium, das ein Beta-Lactamasen-Substrat, insbesondere ein Beta-Lactam, mit elektrochemischen Eigenschaften enthält,
(b) Anwendung einer amperometrischen Messung jeweils an dem am Ende von Schritt (a) erhaltenen Medium und an einer Negativkontrolle,
(c) Bestimmung des Vorhandenseins von Beta-Lactamasen produzierenden Bakterien durch Vergleich des Wertes der Intensität des der Oxidation des hydrolysierten Substrats, insbesondere des hydrolysierten Beta-Lactams, entsprechenden anodischen Stroms, die für das vorgenannte, am Ende von Schritt (a) erhaltene Medium gemessen wurde, mit dem Wert der Intensität des anodischen Stroms, die für die Negativkontrolle gemessen wurde.

5. Verfahren nach Anspruch 4 zur Bestimmung und Quantifizierung von Beta-Lactamasen produzierenden Bakterien in einer Probe, die möglicherweise solche Bakterien enthält, wobei das Verfahren ferner nach Schritt (c) einen Schritt (d) umfasst, der darin besteht, den Wert der Intensität des anodischen Stroms, der für das am Ende von Schritt (a) erhaltene Medium gemessen wurde, mit einer unter den gleichen Bedingungen erstellten Kalibrierungskurve zu vergleichen.

6. Verfahren nach einem der Ansprüche 1 bis 3 zum zusätzlichen Unterscheiden des Typs von Beta-Lactamasen, ausgewählt aus Penicillinasen, Beta-Lactamasen mit erweitertem Spektrum, induzierbaren Cephalosporinasen, hyperproduzierten Cephalosporinasen und Carbapenemasen, die von den genannten Bakterien produziert werden, in einer Probe, die möglicherweise solche enthält, wobei das Verfahren die folgenden Schritte umfasst:
(a) parallele Inkubation einer Fraktion der Probe in:
einem Kulturmedium A, einem Kulturmedium B und einem Kulturmedium C:
- wobei das Kulturmedium A ein Basiskulturmedium ist,
- wobei das Kulturmedium B ein mit einem Cephalosporin der 3. Generation ergänztes Basiskulturmedium ist, und
- wobei das Kulturmedium C ein Basiskulturmedium ist, das mit einem Cephalosporin und einem Penicillinase-Inhibitor ergänzt ist, und
optional in einem Kulturmedium B', einem Kulturmedium C', einem Kulturmedium D, einem Kulturmedium E:
- wobei das Kulturmedium B' ein Basiskulturmedium ist, das mit einem Cephalosporin der 3. Generation ergänzt ist, das sich von dem in Medium B enthaltenen unterscheidet;
- wobei das Kulturmedium C' ein Kulturmedium B' ist, das mit einem Penicillinase-Inhibitor ergänzt ist,
- wobei das Kulturmedium D ein Basiskulturmedium ist, das mit einem Cephalosporin und einem Cephalosporinase-Inhibitor ergänzt ist, und
- wobei das Kulturmedium E ein Basiskulturmedium ist, das mit einem Carbapenem ergänzt ist,
(b) parallele Inkubation der am Ende der Inkubation von Schritt (a) erhaltenen Kulturmedien in einem Nitrocefin enthaltenden Medium.

7. Verfahren nach Anspruch 6 zur Bestimmung des Vorhandenseins von Beta-Lactamasen mit erweitertem Spektrum produzierenden Bakterien, wobei das Verfahren die folgenden Schritte umfasst:
(a) parallele Inkubation einer Fraktion der genannten Probe in einem Kulturmedium A, einem Kulturmedium B und einem Kulturmedium C, und optional in einem Kulturmedium B' und einem Kulturmedium C', wie in Anspruch 6 definiert,
(b) parallele Inkubation der am Ende der Inkubation von Schritt (a) erhaltenen Kulturmedien in einem Nitrocefin enthaltenden Medium.

8. Verfahren nach Anspruch 6, zur Bestimmung und Unterscheidung von Beta-Lactamasen produzierenden Bakterien in einer Probe, die möglicherweise solche enthält, umfassend die folgenden Schritte:
(a) Parallele Inkubation einer Fraktion der Probe in einem Kulturmedium A, einem Kulturmedium B, einem Kulturmedium C, einem Kulturmedium D, einem Kulturmedium E, optional einem Medium B' und einem Medium C', wie in Anspruch 6 definiert,
(b) parallele Inkubation der vorgenannten, am Ende der Inkubation von Schritt (a) erhaltenen Kulturmedien in einem Nitrocefin enthaltenden Medium,
(c) Anwendung einer amperometrischen Messung jeweils auf die am Ende von Schritt (b) erhaltenen Medien und auf eine Negativkontrolle,
(d) Bestimmung des Vorhandenseins von Beta-Lactamasen produzierenden Bakterien in der Probe durch Vergleich des Wertes der Intensität des der Oxidation von hydrolysiertem Nitrocefin entsprechenden anodischen Stroms, der für die im Kulturmedium A kultivierte Fraktion erhalten wurde, mit dem Wert der Intensität des Stroms, der für die Negativkontrolle erhalten wurde, und
(e) Unterscheidung des Typs der von den genannten Bakterien in der genannten Probe produzierten Beta-Lactamasen durch Vergleich der jeweiligen Werte der Intensität des genannten anodischen Stroms, die für die parallel in den Kulturmedien A, B, C, D und E, gegebenenfalls B' und C', kultivierten Fraktionen erhalten wurden, mit den jeweiligen für einen Referenzbakterienstamm erhaltenen Werten.

9. Verfahren nach Anspruch 8, ferner umfassend einen Schritt (f) nach Schritt (e) zur Quantifizierung von Bakterien, die die in Schritt (e) unterschiedene Beta-Lactamase produzieren, durch Vergleich des Wertes der Intensität des anodischen Stroms, der dem durch die Beta-Lactamase hydrolysierten Nitrocefin entspricht, mit einer unter den gleichen Bedingungen erstellten Eichkurve.

10. Verfahren nach einem der Ansprüche 6, 8-9, zur Unterscheidung des Typs von Beta-Lactamasen und optional zur Spezifizierung der Unterfamilie von Carbapenemasen, die von den vorgenannten Bakterien produziert werden, in einer Probe, die möglicherweise solche enthält, wobei das Verfahren die folgenden Schritte umfasst:
(a) parallele Inkubation einer Fraktion der genannten Probe in einem Kulturmedium A, einem Kulturmedium B, einem Kulturmedium C, einem Kulturmedium D, einem Kulturmedium E und einem Kulturmedium F, sowie optional einem Kulturmedium B' und einem Kulturmedium C',
wobei die Medien A, B, B', C, C', D, E wie in Anspruch 6 definiert sind, wobei das Kulturmedium F ein Basiskulturmedium ist, das mit einem Carbapenem und einem für eine Carbapenemasen-Unterfamilie spezifischen Inhibitor ergänzt ist;
(b) parallele Inkubation der am Ende der Inkubation von Schritt (a) erhaltenen Kulturmedien in einem Nitrocefin enthaltenden Medium,
(c) Anwendung einer amperometrischen Messung jeweils auf die oben genannten, am Ende von Schritt (b) erhaltenen Medien und auf eine Negativkontrolle,
(d) Bestimmung des Vorhandenseins von Beta-Lactamasen produzierenden Bakterien in der genannten Probe durch Vergleich des Wertes der Intensität des der Oxidation von hydrolysiertem Nitrocefin entsprechenden anodischen Stroms, der für die im Kulturmedium A kultivierte Fraktion erhalten wurde, mit dem für die Negativkontrolle erhaltenen Wert der Intensität des Stroms,
(e) Unterscheidung des Typs der von den Bakterien in der Probe produzierten Beta-Lactamasen durch Vergleich der jeweiligen Werte der Intensität des anodischen Stroms, die für die Fraktionen erhalten wurden, die parallel in den Kulturmedien A, B, C, D, E und F und gegebenenfalls den Kulturmedien B' und C' kultiviert wurden, mit den jeweiligen Werten, die für einen Referenzbakterienstamm erhalten wurden.

11. Verfahren nach einem der Ansprüche 1 bis 10, **dadurch gekennzeichnet, dass** die Intensität des anodischen Stroms, der der Oxidation von hydrolysiertem Nitrocefin in einem Puffer mit neutralem pH-Wert entspricht, in einem Bereich von Potentialen zwischen + 0,1 V und + 0,5 V gegen Ag/AgCl, insbesondere zwischen + 0,2 V und + 0,4 V gegen Ag/AgCl, insbesondere zwischen + 0,23 V und + 0,33 V gegen Ag/AgCl, gemessen wird.

12. Verfahren nach einem der Ansprüche 6 bis 10, **dadurch gekennzeichnet, dass** ein Cephalosporin der 3. Generation aus der Gruppe ausgewählt ist, die Cefotaxim, Ceftazidim und Ceftriaxon umfasst; dass ein Penicillinase-Inhibitor Clavulansäure, Tazobactam oder Sulbactam ist; dass der Cephalosporinase-Inhibitor Cloxacillin ist; dass ein Carbapenem Ertapenem, Imipenem oder Meropenem ist; dass ein Carbapenemase-Inhibitor ausgewählt ist aus EDTA, Mercaptoessigsäure, Boronsäure oder Clavulansäure.

13. Verfahren nach einem der Ansprüche 1 bis 5, **dadurch gekennzeichnet, dass** das Verfahren verwendet wird, um in-vitro das Vorhandensein von Bakterien zu bestimmen und zu quantifizieren, die gegen ein Cephalosporin der 3. Generation resistent sind, und dass das in Schritt (a) verwendete Medium ein Beta-Lactam enthält, das in der Regel nur durch Beta-Lactamasen mit erweitertem Spektrum, hyperproduzierte Cephalosporinasen und Carbapenemasen hydrolysiert wird.

14. Verfahren nach einem der Ansprüche 1 bis 5, **dadurch gekennzeichnet, dass** das Verfahren verwendet wird, um in-vitro das Vorhandensein von Carbapenemaseproduzierenden Bakterien zu bestimmen und zu quantifizieren, und dass das in Schritt (a) verwendete Medium ein Substrat enthält, das in der Regel nur von Carbapenemasen hydrolysiert wird.

15. Verfahren nach einem der Ansprüche 1 bis 14, **dadurch gekennzeichnet, dass** die genannte Probe aus einer biologischen Probe, einer Probe aus der Umwelt oder einer Lebensmittelprobe ausgewählt ist.

## Claims

1. Method for *in-vitro* determination of the presence of bacteria producing beta-lactamases, in a sample that may contain said bacteria, said method comprising the following steps:
(a) incubating said sample in a medium containing a substrate of the beta-lactamases, having electrochemical properties, in particular a beta-lactam antibiotic having electrochemical properties, and
(b) applying a means of electrochemical analysis in order to determine the presence of the substrate of the beta-lactamases after having been hydrolyzed.

2. Method according to claim 1, **characterized in that** said means of electrochemical analysis is a potentiometric measurement, an impedance measurement, a coulometric measurement or an amperometric measurement.

3. Method according to claim 1 or 2, **characterized in that** said substrate of the beta-lactamases is a beta-lactam antibiotic, in particular selected from the group comprising nitrocefin, the compound HMRZ-86 (E isomer of (6R,7R)-trifluoroacetate 7-[[2-(2-amino-4-thiazolyl)-2-[(1-carboxy-1-methylethoxy)imino]acetyl]amino]-1-aza-3-[2-(2,4-dinitrophenyl)ethenyl]-8-oxo-5-thiabicyclo[4.2.0]oct-2-ene-2-carboxylic acid), or the substrate (reagent R2) of the β CARBA^{™} kit.

4. Method according to any one of claims 1 to 3, **characterized in that** said method comprises the following steps:
(a) incubating a sample that may contain said bacteria in a medium containing a substrate of the beta-lactamases, in particular a beta-lactam antibiotic, having electrochemical properties,
(b) applying an amperometric measurement to the aforesaid medium obtained at the end of step (a) and to a negative control, respectively,
(c) determining the presence of the beta-lactamase-producing bacteria by comparing the value of the intensity of the anodic current corresponding to oxidation of the hydrolyzed substrate, in particular of the hydrolyzed beta-lactam antibiotic, measured for the aforesaid medium obtained at the end of step (a), with the value of the intensity of the anodic current measured for the negative control.

5. Method according to claim 4 in order to determine and quantify the beta-lactamase-producing bacteria in a sample that may contain said bacteria, said method additionally comprising, after step (c), a step (d) consisting of comparing the value of the intensity of the anodic current measured for said medium obtained at the end of step (a) with a calibration curve established under the same conditions.

6. Method according to any one of claims 1 to 3 making it possible in addition to distinguish the type of beta-lactamases selected from penicillinases, extended-spectrum beta-lactamases, inducible cephalosporinases, hyperproduced cephalosporinases and carbapenemases, produced by the aforesaid bacteria in a sample that may contain them, said method comprising the following steps:
(a) incubating, in parallel, a fraction of the aforesaid sample in:
a culture medium A, a culture medium B and a culture medium C:
- culture medium A being a basic culture medium,
- culture medium B being a basic culture medium supplemented with a third-generation cephalosporin, and
- culture medium C being a basic culture medium supplemented with a cephalosporin and a penicillinase inhibitor, and
optionally in a culture medium B', a culture medium C', a culture medium D, a culture medium E:
- culture medium B' being a basic culture medium supplemented with a third-generation cephalosporin different from that present in medium B;
- culture medium C' being culture medium B' supplemented with a penicillinase inhibitor;
- culture medium D being a basic culture medium supplemented with a cephalosporin and a cephalosporinase inhibitor, and
- culture medium E being a basic culture medium supplemented with a carbapenem,
(b) incubating, in parallel, the aforesaid culture media obtained at the end of the incubation in step (a) in a medium containing nitrocefin.

7. Method according to claim 6 in order to determine the presence of the bacteria producing an extended-spectrum beta-lactamase, said method comprising the following steps:
(a) incubating, in parallel, a fraction of the aforesaid sample in a culture medium A, a culture medium B and a culture medium C, and optionally in a culture medium B' and a culture medium C', as defined according to claim 6,
(b) incubating, in parallel, the aforesaid culture media obtained at the end of the incubation in step (a) in a medium containing nitrocefin.

8. Method according to claim 6, in order to determine and distinguish the beta-lactamase-producing bacteria in a sample that may contain them, comprising the following steps:
(a) incubating, in parallel, a fraction of the aforesaid sample in a culture medium A, a culture medium B, a culture medium C, a culture medium D, a culture medium E, optionally a medium B' and a medium C', as defined according to claim 6,
(b) incubating, in parallel, the aforesaid culture media obtained at the end of the incubation in step (a) in a medium containing nitrocefin,
(c) applying an amperometric measurement to the aforesaid media obtained at the end of step (b) and to a negative control, respectively,
(d) determining the presence of beta-lactamase-producing bacteria in said sample by comparing the value of the intensity of the anodic current corresponding to the oxidation of hydrolyzed nitrocefin obtained for the fraction cultured in culture medium A with the value of the intensity of the current obtained for the negative control, and
(e) distinguishing the type of beta-lactamases produced by the aforesaid bacteria in said sample, by comparing the respective values of the intensity of the aforesaid anodic current obtained for the fractions cultured in parallel in culture media A, B, C, D and E, optionally B' and C' with the respective values obtained for a reference bacterial strain.

9. Method according to claim 8, further comprising a step (f) after step (e) making it possible to quantify beta-lactamase-producing bacteria distinguished in step (e) by comparing the value of the intensity of the anodic current corresponding to nitrocefin hydrolyzed by said beta-lactamase with a calibration curve established under the same conditions.

10. Method according to any one of claims 6, 8-9, for distinguishing the type of beta-lactamases and optionally defining the subfamily of carbapenemases produced by the aforesaid bacteria in a sample that may contain them, said method comprising the following steps:
(a) incubating, in parallel, a fraction of the aforesaid sample in a culture medium A, a culture medium B, a culture medium C, a culture medium D, a culture medium E, and a culture medium F, and optionally a culture medium B' and a culture medium C',
the media A, B, B', C, C', D, E being as defined according to claim 6,
culture medium F being a basic culture medium supplemented with a carbapenem and an inhibitor specific to a subfamily of carbapenemases;
(b) incubating, in parallel, the aforesaid culture media obtained at the end of the incubation in step (a) in a medium containing nitrocefin,
(c) applying an amperometric measurement to the aforesaid media obtained at the end of step (b) and to a negative control, respectively,
(d) determining the presence of beta-lactamase-producing bacteria in said sample by comparing the value of the intensity of the anodic current corresponding to the oxidation of hydrolyzed nitrocefin obtained for the fraction cultured in culture medium A with the value of the intensity of the current obtained for the negative control,
(e) distinguishing the type of beta-lactamases produced by the aforesaid bacteria in said sample, by comparing the respective values of the intensity of the aforesaid anodic current obtained for the fractions cultured in parallel in culture media A, B, C, D, E and F, and optionally the culture media B' and C', with the respective values obtained for a reference bacterial strain.

11. Method according to any one of claims 1 to 10, **characterized in that** the intensity of the anodic current corresponding to the oxidation of hydrolyzed nitrocefin in a buffer of neutral pH is measured in a potential range comprised between +0.1 V and +0.5 V *vs.* Ag/AgCl, in particular between +0.2 V and +0.4 V *vs.* Ag/AgCl, more particularly between +0.23V and +0.33V *vs*. Ag/AgCl.

12. Method according to any one of claims 6 to 10, **characterized in that** a third-generation cephalosporin is selected from the group comprising cefotaxime, ceftazidime and ceftriaxone; that a penicillinase inhibitor is clavulanic acid, tazobactam or sulbactam; that said cephalosporinase inhibitor is cloxacillin; that a carbapenem is ertapenem, imipenem or meropenem; that a carbapenemase inhibitor is selected from EDTA, mercaptoacetic acid, boronic acid, or clavulanic acid.

13. Method according to any one of claims 1 to 5, **characterized in that** said method is used in order to determine and quantify *in vitro* the presence of bacteria resistant to a third-generation cephalosporin and that the medium used in step (a) contains a betalactam antibiotic only hydrolyzed by extended-spectrum beta-lactamases, hyperproduced cephalosporinases and carbapenemases.

14. Method according to any one of claims 1 to 5, **characterized in that** said method is used in order to determine and quantify *in vitro* the presence of carbapenemase-producing bacteria and that the medium used in step (a) contains a substrate that is only hydrolyzed by the carbapenemases.

15. Method according to any one of claims 1 to 14, **characterized in that** said sample is selected from a biological sample, a sample of environmental origin, or a food sample.
